# EUROPEAN PATENT APPLICATION

(11) **EP 4 332 542 A1**
(43) Date of publication of application: **06.03.2024**
(21) Application number: 22795740.4
(22) Date of filing: 25.04.2022
(51) Int. Cl.: G01N 1/10, A61B 34/37, B25J 3/00

(54) **ROBOT SYSTEM**

(30) Priority: 26.04.2021 JP 2021074360
(71) Applicant: KAWASAKI JUKOGYO KABUSHIKI KAISHA, Kobe-shi, Hyogo 650-8670 (JP)
(72) Inventor: KOKUSHI, Hiroki, Hyogo 650-8670 (JP); RYU, Hideyuki, Hyogo 650-8670 (JP)
(74) Representative: Hoffmann Eitle
(86) International application number: PCT/JP2022/018782
(87) International publication number: WO 2022/230827

(57) **Abstract**

A robot system (100) includes a slave robot (10), a master robot (20), and a controller (50) configured or programmed to control straight-ahead movement of a hand (12) and at least a portion of rotational movement of the hand independently of each other by dividing an operation system when a procedure is performed on a person to be treated using a treatment member (101) held by the hand.

## Description

### Technical Field

The present disclosure relates to a robot system, and more particularly, it relates to a robot system that performs a procedure such as collecting a specimen from a person to be treated.

### Background Art

Conventionally, a specimen collection box for collecting a specimen from a person to be treated is known. Such a specimen collection box is disclosed in Japanese Utility Model Registration No. 3228999. Japanese Utility Model Registration No. 3228999 discloses a specimen collection box including a specimen collection main body box and a pair of protective gloves provided on the specimen collection main body box. In this specimen collection box, an operation person who collects a specimen is placed in the specimen collection main body box and collects the specimen from a person to be treated through the pair of protective gloves.

### Prior Art

### Patent Document

Patent Document 1: Japanese Utility Model Registration No. 3228999

### Summary of the Invention

However, in the technology described in Japanese Utility Model Registration No. 3228999, the operation person is placed in the specimen collection main body box and collects the specimen from the person to be treated through the pair of protective gloves, and thus the operation person needs to be positioned close to the person to be treated. Furthermore, the operation person is placed in the specimen collection main body box, and thus it is necessary to take air into the specimen collection main body box. Therefore, the operation person has a high risk of being infected when collecting the specimen from the person to be treated.

The present disclosure is intended to solve the above problem. The present disclosure aims to provide a robot system capable of reducing the risk of infection from a person to be treated to an operation person.

In order to attain the aforementioned object, a robot system according to a first aspect of the present disclosure includes a slave robot including a hand to hold a treatment member and operable to perform a procedure on a person to be treated using the treatment member held by the hand, a master robot operated by an operation person to remotely control the slave robot, and a controller configured or programmed to control straight-ahead movement of the hand and at least a portion of rotational movement of the hand independently of each other by dividing an operation system when the procedure is performed on the person to be treated using the treatment member held by the hand. The term "procedure" indicates a broader concept including collecting a specimen from the person to be treated, performing an examination on the person to be treated, performing surgery on the person to be treated, etc.

As described above, the robot system according to the first aspect of the present disclosure includes the slave robot to perform the procedure on the person to be treated using the treatment member, and the master robot operated by the operation person to remotely control the slave robot. Accordingly, the operation person can perform the procedure on the person to be treated by remotely controlling the slave robot using the master robot, and thus the risk of infection from the person to be treated to the operation person can be reduced. Furthermore, the robot system includes the controller configured or programmed to control the straight-ahead movement of the hand and at least the portion of the rotational movement of the hand independently of each other by dividing the operation system when the procedure is performed on the person to be treated using the treatment member held by the hand. Accordingly, unintended and erroneous movement of the hand caused by an erroneous operation can be reduced or prevented unlike a case in which the hand of the slave robot is moved straight ahead and rotationally by one operation system. Consequently, it is possible to improve the operability when the hand of the slave robot is remotely controlled by the master robot to perform the procedure on the person to be treated.

In order to attain the aforementioned object, a robot system according to a second aspect of the present disclosure includes a slave robot including a hand to hold a treatment member and operable to perform a procedure on a person to be treated using the treatment member held by the hand, a master robot operated by an operation person to remotely control the slave robot, and a controller configured or programmed to control straight-ahead movement of the hand and at least a portion of rotational movement of the hand independently of each other by dividing an operation system by a plurality of operators, a switch, or rotational movement automation when the procedure is performed on the person to be treated using the treatment member held by the hand.

As described above, the robot system according to the second aspect of the present disclosure includes the slave robot to perform the procedure on the person to be treated using the treatment member, and the master robot operated by the operation person to remotely control the slave robot. Accordingly, the operation person can perform the procedure on the person to be treated by remotely controlling the slave robot using the master robot, and thus the risk of infection from the person to be treated to the operation person can be reduced. Furthermore, the robot system includes the controller configured or programmed to control the straight-ahead movement of the hand and at least the portion of the rotational movement of the hand independently of each other by dividing the operation system by the plurality of operators, the switch, or the rotational movement automation when the procedure is performed on the person to be treated using the treatment member held by the hand. Accordingly, unintended and erroneous movement of the hand caused by an erroneous operation can be reduced or prevented unlike a case in which the hand of the slave robot is moved straight ahead and rotationally by one operation system. Consequently, it is possible to provide the robot system capable of improving the operability when the hand of the slave robot is remotely controlled by the master robot to perform the procedure on the person to be treated.

According to the present disclosure, as described above, the risk of infection from the person to be treated to the operation person can be reduced.

### Brief Description of the Drawings

FIG. 1 is a diagram showing a specimen collection robot system according to a first embodiment.
FIG. 2 is a diagram showing a slave robot according to the first embodiment.
FIG. 3 is a diagram showing the slave robot and a subject according to the first embodiment.
FIG. 4 is a diagram for illustrating specimen collection by a hand according to the first embodiment.
FIG. 5 is a diagram for illustrating movement of the hand according to the first embodiment.
FIG. 6 is a diagram showing a first operating device of a master robot according to the first embodiment.
FIG. 7 is a diagram showing a second operating device of the master robot according to the first embodiment.
FIG. 8 is a diagram for illustrating control of a controller according to the first embodiment.
FIG. 9 is a diagram for illustrating conversion of arcuate movement of the hand into linear movement according to the first embodiment.
FIG. 10 is a flowchart for illustrating a control process for specimen collection work according to the first embodiment.
FIG. 11 is a flowchart for illustrating a process of conversion of the arcuate movement into the linear movement according to the first embodiment.
FIG. 12 is a diagram showing a specimen collection robot system according to a second embodiment.
FIG. 13 is a diagram showing a master robot according to the second embodiment.
FIG. 14 is a diagram for illustrating control of a controller according to the second embodiment.
FIG. 15 is a flowchart for illustrating a control process for specimen collection work according to the second embodiment.
FIG. 16 is a diagram showing a specimen collection robot system according to a third embodiment.
FIG. 17 is a diagram showing a master robot according to the third embodiment.
FIG. 18 is a diagram for illustrating control of a controller according to the third embodiment.
FIG. 19 is a flowchart for illustrating a control process for specimen collection work according to the third embodiment.
FIG. 20 is a diagram showing a specimen collection robot system according to a fourth embodiment.
FIG. 21 is a diagram for illustrating control of a controller according to the fourth embodiment.
FIG. 22 is a flowchart for illustrating a control process for specimen collection work according to the fourth embodiment.
FIG. 23 is a diagram showing a specimen collection robot system according to a fifth embodiment.
FIG. 24 is a diagram showing a master robot according to the fifth embodiment.
FIG. 25 is an enlarged view showing the vicinity of a handle of the master robot according to the fifth embodiment.
FIG. 26 is a diagram for illustrating control of a controller according to the fifth embodiment.
FIG. 27 is a flowchart for illustrating a control process for specimen collection work according to the fifth embodiment.
FIG. 28 is a diagram showing a specimen collection robot system according to a sixth embodiment.
FIG. 29 is an enlarged view showing the vicinity of a handle of a master robot according to the sixth embodiment.
FIG. 30 is a diagram showing a slave robot according to a modified example.
FIG. 31 is a diagram showing ureteroscopic surgery performed by operating the slave robot according to the modified example.
FIG. 32 is a diagram for illustrating movement of a hand according to the modified example.

### Modes for Carrying Out the Invention

### First Embodiment

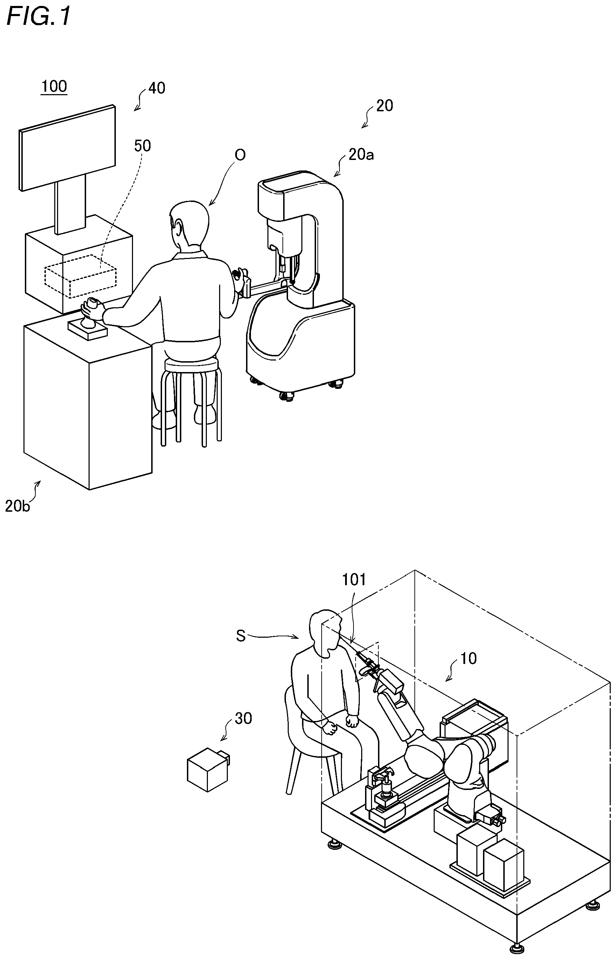

As shown in FIG. 1, a specimen collection robot system 100 according to a first embodiment includes a slave robot 10, a master robot 20, an imager 30, a display 40, and a controller 50. The specimen collection robot system 100 is an example of a "robot system" in the claims.

As shown in FIGS. 1 to 4, the slave robot 10 performs a procedure to collect a specimen from a subject S using a specimen collection member 101. The specimen collection member 101 is a sterile swab, for example. The sterile swab has a stick shape. The slave robot 10 inserts the specimen collection member 101 into the nasal cavity of the subject S, for example, and collects the specimen (nasopharyngeal swab) from the nasopharynx of the subject S by the inserted specimen collection member 101. The slave robot 10 may insert the specimen collection member 101 into the oral cavity of the subject S to collect the specimen. A virus test (a test for new coronavirus, for example) such as a PCR (polymerase chain reaction) test is performed on the collected specimen. In the specimen collection robot system 100, a person in charge of specimen collection, such as a doctor, does not need to face the subject S to collect the specimen, and thus it is possible to isolate the person in charge of specimen collection from the risk of infection. The specimen collection member 101 is an example of a "treatment member" in the claims. The subject S is an example of a "person to be treated" in the claims.

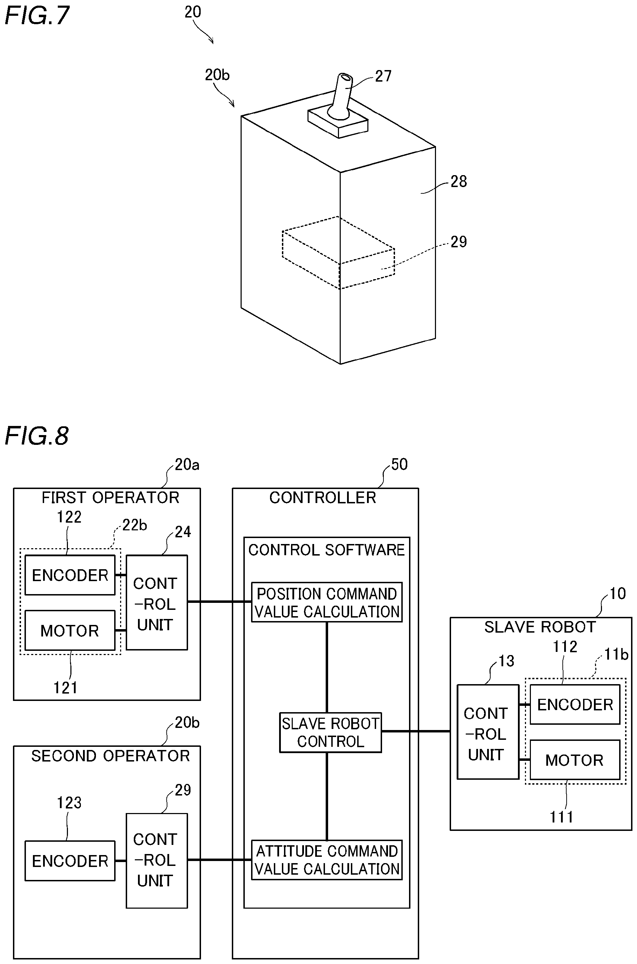

As shown in FIG. 1, the slave robot 10 is a vertical articulated robot. The slave robot 10 includes an arm 11 and a hand 12 attached to a tip end of the arm 11. The arm 11 includes a plurality of links 11a. The plurality of links 11a are connected to each other by joints 11b. A plurality of joints 11b are provided. Each of the plurality of joints 11b includes a motor 111 (servomotor) (see FIG. 8) as a drive and an encoder 112 (see FIG. 8) as a position detector. The hand 12 holds the specimen collection member 101. The hand 12 grips and holds the specimen collection member 101 with a chuck mechanism, for example.

The slave robot 10 is housed in a housing 60. A control unit 13 that controls the slave robot 10 is provided inside the housing 60.

A shielding plate 61 is arranged between the slave robot 10 and the subject S. The shielding plate 61 is installed on the housing 60. The shielding plate 61 is made of a colorless and transparent member such as a glass plate or an acrylic plate, and includes an opening 61a.

The opening 61a is provided at a position corresponding to the nasal cavity of the subject S, and the size of the opening 61a corresponds to a size including the nose and mouth of the subject S.

A positioner 70 is installed between the shielding plate 61 and the subject S. The positioner 70 includes a contacted portion 71 and a chin rest 72.

When the subject S brings the contacted portion 71 into contact with their forehead and places their chin on the chin rest 72, the positioner 70 positions the nasal cavity of the subject S within a preset range (opening 61a). Thus, the nasal cavity of the subject S can be easily positioned.

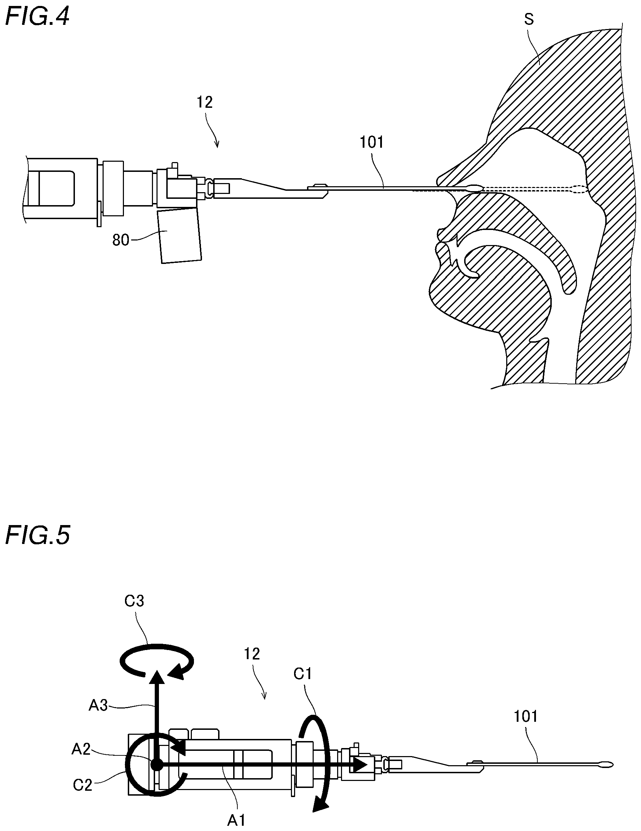

An imager 80 is provided on the arm 11 or the hand 12 (on a lower portion of the hand 12 in FIG. 4) to image the subject S and the specimen collection member 101. The imager 80 is a camera that captures a two-dimensional image. The imager 80 may be a camera that captures a three-dimensional image. The imager 80 images the entire face of the subject S including the nasal cavity from the front side of the subject S. The imager 80 images the specimen collection member 101 held by the hand 12.

As shown in FIG. 5, the hand 12 is operated in a plurality of directions. Specifically, the hand 12 is moved straight ahead (Cartesian coordinate motion) in a forward-rearward direction (A1 direction), a right-left direction (A2 direction), and an upward-downward direction (A3 direction) by movement of the plurality of joints 11b of the arm 11, and is rotationally moved in a first rotation direction (C1 direction) about a first rotation axis extending in the forward-rearward direction, a second rotation direction (C2 direction) about a second rotation axis extending in the right-left direction, and a third rotation direction (C3 direction) about a third rotation axis extending in the upward-downward direction. The forward-rearward direction, the right-left direction, the upward-downward direction, the first rotation direction, the second rotation direction, and the third rotation direction are directions with respect to the hand 12. The straight-ahead movement of the hand 12 in the forward-rearward direction substantially matches straight-ahead movement of the specimen collection member 101 in an axial direction. Furthermore, the rotational movement of the hand 12 in the first rotation direction substantially matches rotational movement of the specimen collection member 101 about the axis. Moreover, the rotational movement of the hand 12 in the first rotation direction (C1 direction) is performed when the specimen is scraped at a specimen collection position (nasopharynx) of the subject S.

As shown in FIGS. 1, 6, and 7, the master robot 20 remotely controls the slave robot 10. Specifically, the master robot 20 remotely controls the slave robot 10 by being operated by an operation person 0 such as a doctor. The master robot 20 outputs an operation command based on an operation of the operation person O. The slave robot 10 performs an action corresponding to the operation of the operation person 0 based on the operation command from the master robot 20.

In this embodiment, the master robot 20 includes a first operator 20a and a second operator 20b provided separately from and independently of the first operator 20a. The straight-ahead movement of the hand 12 is assigned to the first operator 20a. Specifically, the straight-ahead movement of the hand 12 in the forward-rearward direction (A1 direction), the right-left direction (A2 direction), and the upward-downward direction (A3 direction) is assigned to the first operator 20a. The rotational movement of the hand 12 is assigned to the second operator 20b. Specifically, the rotational movement of the hand 12 in the first rotation direction (C1 direction), the second rotation direction (C2 direction), and the third rotation direction (C3 direction) is assigned to the second operator 20b. The first operator 20a is an example of a "first operator" or a "fifth operator" in the claims. The second operator 20b is an example of a "second operator" in the claims.

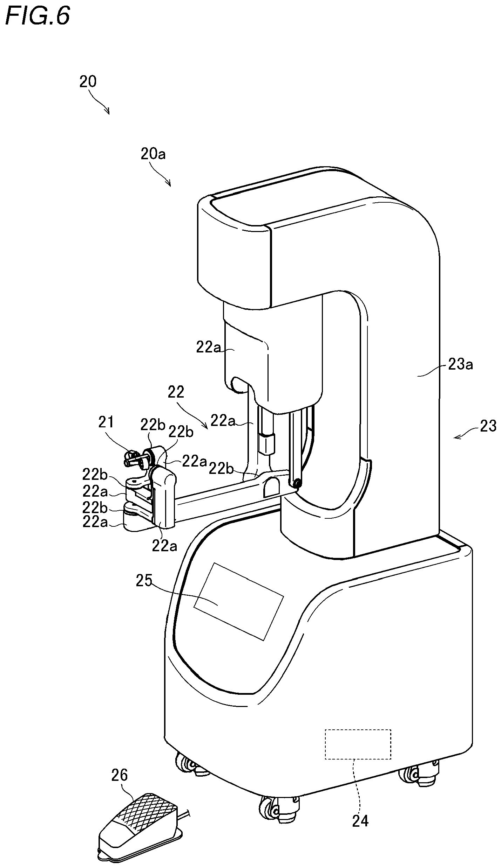

As shown in FIG. 6, the first operator 20a includes a movable operation handle 21 and an arm 22 that movably supports the operation handle 21. The operation handle 21 is provided to operate the slave robot 10. Specifically, the operation handle 21 is provided to remotely operate the hand 12 of the slave robot 10 that holds the specimen collection member 101. The operation handle 21 is a stick-shaped grip handle. The operation handle 21 is operated by one hand (the right hand in FIG. 1) of the operation person O. The operation handle 21 is provided to move the hand 12 straight ahead in the forward-rearward direction (A1 direction), the right-left direction (A2 direction), and the upward-downward direction (A3 direction). The arm 22 includes a plurality of links 22a. The plurality of links 22a are connected to each other by joints 22b. A plurality of joints 22b are provided. Each of the plurality of joints 22b includes a motor 121 (servomotor) (see FIG. 8) as a drive and an encoder 122 (see FIG. 8) as a position detector. The operation handle 21 is an example of a "first handle" or a "sixth handle" in the claims.

The first operator 20a also includes a gantry 23. The gantry 23 includes a support column 23a that bends toward the operation person 0 (in a substantially L-shape) on the upper side. A substantially L-shaped portion of the arm 22 is provided below the bent portion of the support column 23a.

A control unit 24 is provided in the gantry 23 to control the first operator 20a. A power supply unit (not shown) etc. is provided in the gantry 23.

An operation panel 25 is provided on the outer surface of the gantry 23. The operation person 0 operates the operation panel 25 such that basic movement instructions, control switching, setting, etc. for the slave robot 10 and the master robot 20 are performed.

The first operator 20a includes a clutch pedal 26. The operation person O depresses the clutch pedal 26 while holding the operation handle 21 such that a clutch operation to not operate the slave robot 10 but operate only the first operator 20a of the master robot 20 is performed.

As shown in FIG. 7, the second operator 20b includes an operation handle 27. The operation handle 27 is provided to operate the slave robot 10. Specifically, the operation handle 27 is provided to remotely operate the hand 12 of the slave robot 10 that holds the specimen collection member 101. The operation handle 27 is a stick-shaped joystick. The operation handle 27 is operated by the other hand (the left hand in FIG. 1) of the operation person O. The operation handle 27 is provided to rotationally move the hand 12 in the first rotation direction (C1 direction), the second rotation direction (C2 direction), and the third rotation direction (C3 direction). The operation handle 27 includes an encoder 123 (see FIG. 8) as a position detector. The operation handle 27 is an example of a "second handle" in the claims.

The second operator 20b also includes a pedestal 28. A control unit 29 is provided in the pedestal 28 to control the second operator 20b.

The hand 12 of the slave robot 10 is moved based on operations on the operation handle 21 of the first operator 20a and the operation handle 27 of the second operator 20b. When the operation handle 21 of the first operator 20a is operated in a direction corresponding to one of the forward-rearward direction (A1 direction), the right-left direction (A2 direction), and the upward-downward direction (A3 direction) of the hand 12, the hand 12 of the slave robot 10 is moved in one of the forward-rearward direction, the right-left direction, and the upward-downward direction. When the operation handle 27 of the second operator 20b is operated in a direction corresponding to one of the first rotation direction (C1 direction), the second rotation direction (C2 direction), and the third rotation direction (C3 direction) of the hand 12, the hand 12 of the slave robot 10 is moved in one of the first rotation direction, the second rotation direction, and the third rotation direction. The first operator 20a and the second operator 20b are operated individually or concurrently.

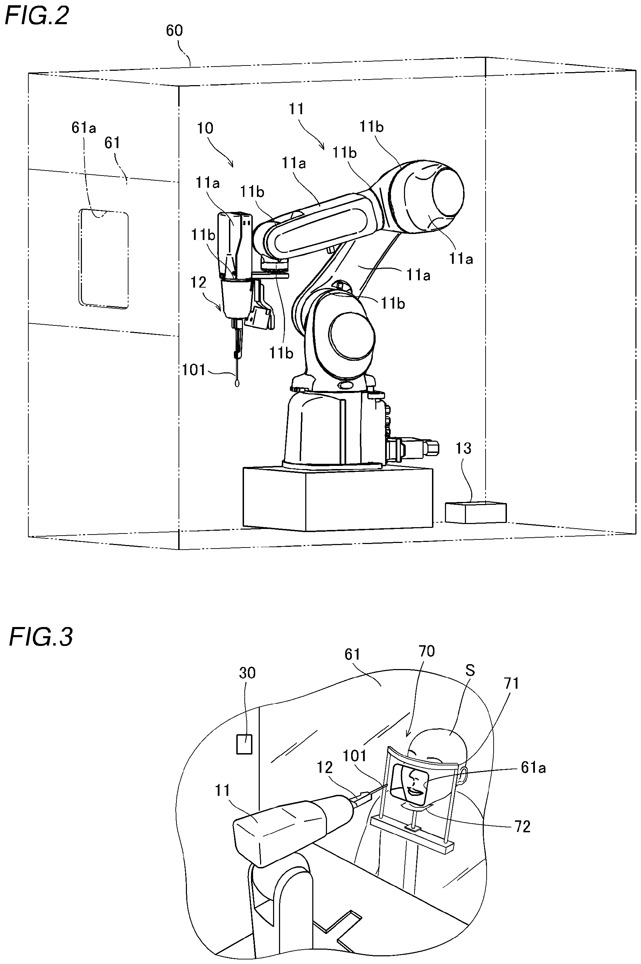

As shown in FIGS. 1 and 3, the imager 30 images the subject S and the specimen collection member 101 from the side. That is, the imager 30 images the side face of the subject S. The imager 30 is a camera that captures a two-dimensional image. The imager 30 images the entire side face of the subject S including the nose from the side of the subject S. The imager 30 images the specimen collection member 101 held by the hand 12. The imager 30 is arranged in the vicinity of the shielding plate 61 and on the subject S side of the shielding plate 61, for example.

As shown in FIG. 1, the display 40 displays an image (video) of the subject S and the specimen collection member 101. The display 40 displays an image captured by the imager 30 and an image captured by the imager 80, for example. The operation person 0 operates the slave robot 10 using the master robot 20 to collect a specimen from the subject S by the specimen collection member 101 while visually recognizing the real-time image of the subject S and the specimen collection member 101 displayed on the display 40. The display 40 includes a liquid crystal monitor, for example.

As shown in FIGS. 1 and 8, the controller 50 controls movement of the slave robot 10 based on an operation on the master robot 20 by the operation person O. The controller 50 includes a computer including a processor such as a CPU and memories such as a RAM and a ROM. Control software for controlling the slave robot 10 is stored in the memory of the controller 50.

In the first embodiment, the controller 50 controls the straight-ahead movement of the hand 12 and at least a portion of the rotational movement of the hand 12 independently of each other by dividing an operation system when a specimen is collected from the subject S by the specimen collection member 101 held by the hand 12. Specifically, when the specimen is collected from the subject S by the specimen collection member 101 held by the hand 12, the controller 50 controls the straight-ahead movement of the hand 12 and at least a portion of the rotational movement of the hand 12 independently of each other by dividing the operation system by a plurality of operators (the first operator 20a and the second operator 20b). Furthermore, the controller 50 controls the straight-ahead movement of the hand 12 and at least the rotational movement of the specimen collection member 101 of the hand 12 independently of each other by dividing the operation system.

In the first embodiment, the controller 50 controls the straight-ahead movement of the hand 12 and at least a portion of the rotational movement of the hand 12 independently of each other by controlling the straight-ahead movement of the hand 12 based on an operation on the first operator 20a by the operation person 0 and at least a portion of the rotational movement of the hand 12 based on an operation on the second operator 20b by the operation person 0 to divide the operation system. Specifically, the controller 50 controls the straight-ahead movement of the hand 12 in the forward-rearward direction (A1 direction), the right-left direction (A2 direction), and the upward-downward direction (A3 direction) based on an operation on the operation handle 21 of the first operator 20a, and controls the rotational movement of the hand 12 in the first rotation direction (C1 direction), the second rotation direction (C2 direction), and the third rotation direction (C3 direction) based on an operation on the operation handle 27 of the second operator 20b.

When the operation handle 21 of the first operator 20a of the master robot 20 is operated by the operation person O, the control unit 24 of the first operator 20a of the master robot 20 outputs the operation amount of the operation handle 21 to the controller 50 based on the detection result of the encoder 122. The controller 50 calculates a position command value for the slave robot 10 based on the operation amount of the operation handle 21 output from the control unit 24. When the operation handle 21 is not operated by the operation person O, the position command value is zero.

When the operation handle 27 of the second operator 20b of the master robot 20 is operated by the operation person O, the control unit 29 of the second operator 20b of the master robot 20 outputs the operation amount of the operation handle 27 to the controller 50 based on the detection result of the encoder 123. The controller 50 calculates an attitude command value for the slave robot 10 based on the operation amount of the operation handle 27 output from the control unit 29. When the operation handle 21 is not operated by the operation person O, the position command value is zero. When the operation handle 27 is not operated by the operation person O, the attitude command value is zero.

The controller 50 calculates a movement command value for the slave robot 10 based on the calculated position command value and attitude command value, and outputs the calculated movement command value to the control unit 13 of the slave robot 10. The control unit 13 of the slave robot 10 controls the operation of the motors 111 of the joints 11b based on the movement command value output from the controller 50. Consequently, the movement of the hand 12 of the slave robot 10 is controlled so as to correspond to operations on the operation handle 21 and the operation handle 27 of the master robot 20.

### Regarding Conversion of Arcuate Movement

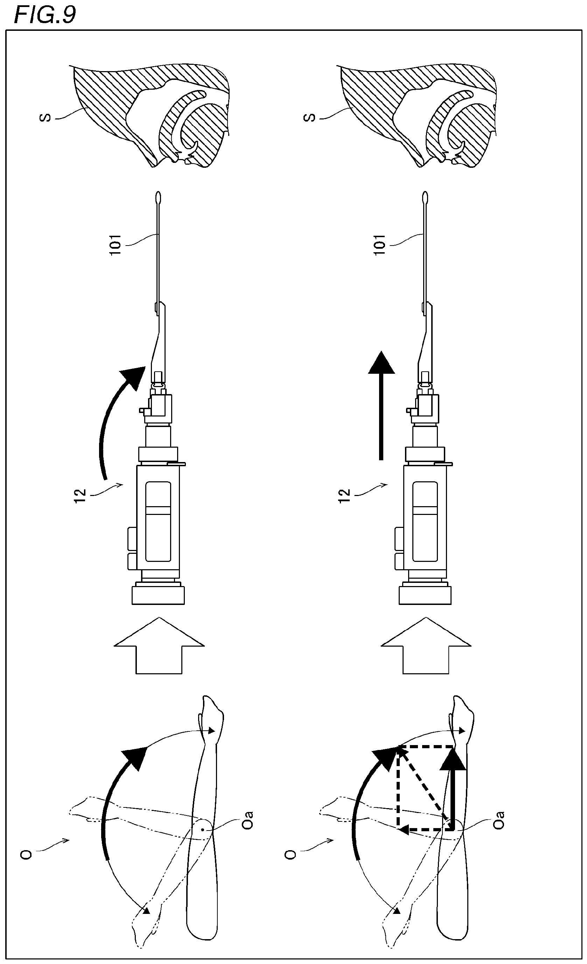

Conversion of the arcuate movement of the hand 12 is now described with reference to FIG. 9.

For example, when the specimen collection member 101 is inserted into the nasal cavity of the subject S, the hand 12 needs to move straight ahead in the forward-rearward direction (A1 direction). When the operation person 0 operates the operation handle 21 with their elbow Oa placed on an elbow rest (not shown), the operation person 0 operates the operation handle 21 by rotating their forearm using their elbow Oa placed on the elbow rest as the center of rotation (fulcrum) even when the operation person 0 tries to operate the operation handle 21 such that the operation handle 21 linearly moves. In this case, the operation handle 21 is operated along an arcuate trajectory by the operation person O, and the hand 12 is unintentionally moved arcuately.

Therefore, in the first embodiment, as shown in a lower portion of FIG. 9, when the operation handle 21 of the first operator 20a is operated along an arcuate trajectory by the operation person O, the controller 50 converts arcuate movement of the hand 12 into linear movement based on an operation on the operation handle 21 by the operation person O. Specifically, the controller 50 converts the arcuate movement of the hand 12 into the linear movement by extracting only an operation for the hand 12 in a specified direction among the forward-rearward direction (A1 direction), the right-left direction (A2 direction), and the upward-downward direction (A3 direction) based on an operation on the operation handle 21 of the first operator 20a by the operation person O.

More specifically, the controller 50 acquires a speed command value for straight-ahead movement of the hand 12 in the forward-rearward direction, a speed command value for straight-ahead movement of the hand 12 in the right-left direction, and a speed command value for straight-ahead movement of the hand 12 in the upward-downward direction. Then, the controller 50 extracts the maximum speed command value from the three acquired speed command values including the speed command value for straight-ahead movement of the hand 12 in the forward-rearward direction, the speed command value for straight-ahead movement of the hand 12 in the right-left direction, and the speed command value for straight-ahead movement of the hand 12 in the upward-downward direction. Then, the controller 50 converts the arcuate movement of the hand 12 into the linear movement (straight-ahead movement with the maximum speed command value) by validating the extracted maximum speed command value and invalidating the speed command values other than the maximum speed command value. The controller 50 invalidates the speed command values other than the maximum speed command value by setting the same to zero or ignoring the same, for example.

### Control Process for Specimen Collection Work

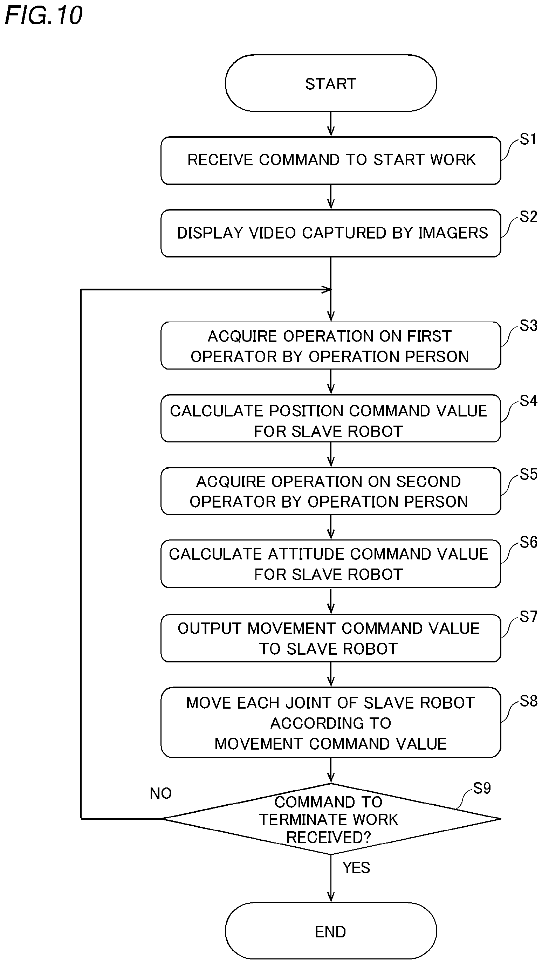

A control process for specimen collection work of the specimen collection robot system 100 according to the first embodiment is now described based on a flowchart with reference to FIG. 10.

As shown in FIG. 10, first, in step S1, a command to start the specimen collection work is received.

Then, in step S2, images (video) captured by the imager 30 and the imager 80 are displayed on the display 40.

Then, in step S3, the operation amount of the operation handle 21 of the first operator 20a by the operation person 0 is acquired.

Then, in step S4, the position command value for the slave robot 10 is calculated based on the operation amount of the operation handle 21 by the operation person 0 acquired in step S3.

Then, in step S5, the operation amount of the operation handle 27 of the second operator 20b by the operation person O is acquired.

Then, in step S6, the attitude command value for the slave robot 10 is calculated based on the operation amount of the operation handle 27 by the operation person 0 acquired in step S5.

Then, in step S7, the movement command value for the slave robot 10 is calculated based on the position command value calculated in step S4 and the attitude command value calculated in step S6, and the calculated movement command value is output to the slave robot 10.

Then, in step S8, the movement of each joint 11b of the slave robot 10 is controlled based on the movement command value output in step S7.

Then, in step S9, it is determined whether or not a command to terminate the specimen collection work has been received. When it is determined that the command to terminate the specimen collection work has not been received, the process returns to step S3. Then, the process operations in step S3 to step S8 are repeated. When it is determined that the command to terminate the specimen collection work has been received, the control process is terminated.

### Process of Conversion of Arcuate Movement

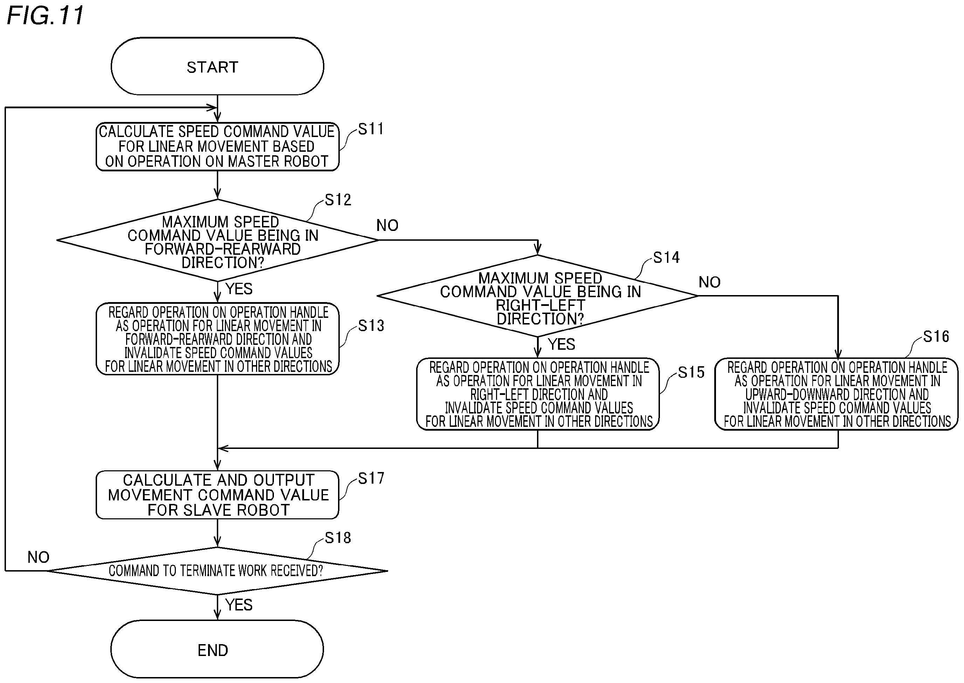

A process of conversion of the arcuate movement of the hand 12 into the linear movement in the specimen collection robot system 100 according to the first embodiment is now described based on a flowchart with reference to FIG. 11.

As shown in FIG. 11, first, in step S11, the three speed command values including the speed command value for straight-ahead movement of the hand 12 in the forward-rearward direction (A1 direction), the speed command value for straight-ahead movement of the hand 12 in the right-left direction (A2 direction), and the speed command value for straight-ahead movement of the hand 12 in the upward-downward direction (A3 direction) are calculated based on an operation on the operation handle 21 of the master robot 20 by the operation person O.

Then, in step S12, it is determined whether or not the maximum speed command value among the three speed command values calculated in step S11 is the speed command value for straight-ahead movement of the hand 12 in the forward-rearward direction (A1 direction). When it is determined that the maximum speed command value among the three speed command values is the speed command value for straight-ahead movement of the hand 12 in the forward-rearward direction, the process advances to step S13.

Then, in step S13, the operation on the operation handle 21 by the operation person 0 is regarded as an operation for straight-ahead movement of the hand 12 in the forward-rearward direction (A1 direction), and the speed command value for straight-ahead movement of the hand 12 in the right-left direction (A2 direction) and the speed command value for straight-ahead movement of the hand 12 in the upward-downward direction (A3 direction) are invalidated. Then, the process advances to step S17.

When it is determined in step S12 that the maximum speed command value among the three speed command values is not the speed command value for straight-ahead movement of the hand 12 in the forward-rearward direction (A1 direction), the process advances to step S14.

Then, in step S14, it is determined whether or not the maximum speed command value among the three speed command values is the speed command value for straight-ahead movement of the hand 12 in the right-left direction (A2 direction). When it is determined that the maximum speed command value among the three speed command values is the speed command value for straight-ahead movement of the hand 12 in the right-left direction, the process advances to step S15.

Then, in step S15, the operation on the operation handle 21 by the operation person 0 is regarded as an operation for straight-ahead movement of the hand 12 in the right-left direction (A2 direction), and the speed command value for straight-ahead movement of the hand 12 in the forward-rearward direction (A1 direction) and the speed command value for straight-ahead movement of the hand 12 in the upward-downward direction (A3 direction) are invalidated. Then, the process advances to step S17.

When it is determined in step S14 that the maximum speed command value among the three speed command values is not the speed command value for straight-ahead movement of the hand 12 in the right-left direction (A2 direction), the process advances to step S16.

Then, in step S16, the operation on the operation handle 21 by the operation person 0 is regarded as an operation for straight-ahead movement of the hand 12 in the upward-downward direction (A3 direction), and the speed command value for straight-ahead movement of the hand 12 in the forward-rearward direction (A1 direction) and the speed command value for straight-ahead movement of the hand 12 in the right-left direction (A2 direction) are invalidated. Then, the process advances to step S17.

Then, in step S17, the movement command value for the slave robot 10 is calculated while any two of the three speed command values are invalidated, and the calculated movement command value is output to the slave robot 10.

Then, in step S18, it is determined whether or not a command to terminate the specimen collection work has been received. When it is determined that the command to terminate the specimen collection work has not been received, the process returns to step S11. Then, the process operations in step S11 to step S17 are repeated as appropriate. When it is determined that the command to terminate the specimen collection work has been received, the control process is terminated.

### Advantages of First Embodiment

According to the first embodiment, the following advantages are achieved.

According to the first embodiment, as described above, the specimen collection robot system 100 includes the slave robot 10 to collect a specimen from the subject S using the specimen collection member 101, and the master robot 20 operated by the operation person 0 to remotely control the slave robot 10. Accordingly, the operation person 0 can collect a specimen from the subject S by remotely controlling the slave robot 10 using the master robot 20, and thus the risk of infection from the subject S to the operation person 0 can be reduced. Furthermore, the specimen collection robot system 100 includes the controller 50 configured or programmed to control the straight-ahead movement of the hand 12 and at least a portion of the rotational movement of the hand 12 independently of each other by dividing the operation system when a specimen is collected from the subject S by the specimen collection member 101 held by the hand 12. Accordingly, unintended movement of the hand 12 caused by an erroneous operation can be reduced or prevented as compared with a configuration in which the hand 12 of the slave robot 10 is moved straight ahead and rotationally by one operation system. Consequently, it is possible to improve the operability when the hand 12 of the slave robot 10 is remotely controlled by the master robot 20 to perform a procedure on the subject S (collect a specimen from the subject S).

According to the first embodiment, as described above, the controller 50 is configured or programmed to control the straight-ahead movement of the hand 12 and at least the rotational movement of the specimen collection member 101 of the hand 12 as the rotational movement of the hand 12 independently of each other by dividing the operation system. Accordingly, when a specimen is scraped from the subject S by the rotational movement of the specimen collection member 101 of the hand 12, unintended and erroneous straight-ahead movement of the hand 12 caused by an erroneous operation can be reduced or prevented. Consequently, it is possible to improve the operability when the hand 12 of the slave robot 10 is remotely controlled by the master robot 20 to collect a specimen from the subject S.

According to the first embodiment, as described above, the master robot 20 includes the first operator 20a to which the straight-ahead movement of the hand 12 is assigned, and the second operator 20b to which at least a portion of the rotational movement of the hand 12 is assigned. Furthermore, the controller 50 is configured or programmed to control the straight-ahead movement of the hand 12 and at least a portion of the rotational movement of the hand 12 independently of each other by controlling the straight-ahead movement of the hand 12 based on an operation on the first operator 20a by the operation person 0 and at least a portion of the rotational movement of the hand 12 based on an operation on the second operator 20b by the operation person O to divide the operation system. Accordingly, the straight-ahead movement of the hand 12 and at least a portion of the rotational movement of the hand 12 can be performed by operating different operators, the first operator 20a and the second operator 20b, and thus erroneous operations for the straight-ahead movement of the hand 12 and at least a portion of the rotational movement of the hand 12 can be easily reduced or prevented. Consequently, unintended and erroneous movement of the hand 12 caused by an erroneous operation can be easily reduced or prevented.

According to the first embodiment, as described above, the first operator 20a includes the operation handle 21 operated by one hand of the operation person 0 to move the hand 12 straight ahead in the forward-rearward direction, the right-left direction, and the upward-downward direction with respect to the hand 12. Furthermore, the second operator 20b includes the operation handle 27 operated by the other hand of the operation person 0 to rotationally move the hand 12 in the first rotation direction about the first rotation axis extending in the forward-rearward direction, the second rotation direction about the second rotation axis extending in the right-left direction, and the third rotation direction about the third rotation axis extending in the upward-downward direction. Moreover, the controller 50 is configured or programmed to control the straight-ahead movement of the hand 12 in the forward-rearward direction, the right-left direction, and the upward-downward direction based on an operation on the operation handle 21, and control the rotational movement of the hand 12 in the first rotation direction, the second rotation direction, and the third rotation direction based on an operation on the operation handle 27. Accordingly, operations for the straight-ahead movement of the hand 12 in the forward-rearward direction, the right-left direction, and the upward-downward direction can be performed by one hand of the operation person O, and operations for the rotational movement of the hand 12 in the first rotation direction, the second rotation direction, and the third rotation direction can be performed by the other hand of the operation person O, and thus erroneous operations for the straight-ahead movement of the hand 12 and the rotational movement of the hand 12 can be more easily reduced or prevented. Thus, unintended and erroneous movement of the hand 12 caused by an erroneous operation can be more easily reduced or prevented.

According to the first embodiment, as described above, the master robot 20 includes the first operator 20a to which the straight-ahead movement of the hand 12 is assigned. The first operator 20a includes the operation handle 21 operated by one hand of the operation person O. The controller 50 is configured or programmed to perform a control to convert the arcuate movement of the hand 12 into the linear movement based on an operation on the operation handle 21 by the operation person 0 when the operation handle 21 is operated along an arcuate trajectory by the operation person O. Accordingly, even when the hand 12 attempts to arcuately move due to the operation of the operation handle 21 of the first operator 20a along an arcuate trajectory by the operation person 0 in spite of the desire to linearly move the hand 12, the arcuate movement of the hand 12 can be converted into linear movement. Consequently, the linear movement of the hand 12 intended by the operation person 0 can be easily performed.

### Second Embodiment

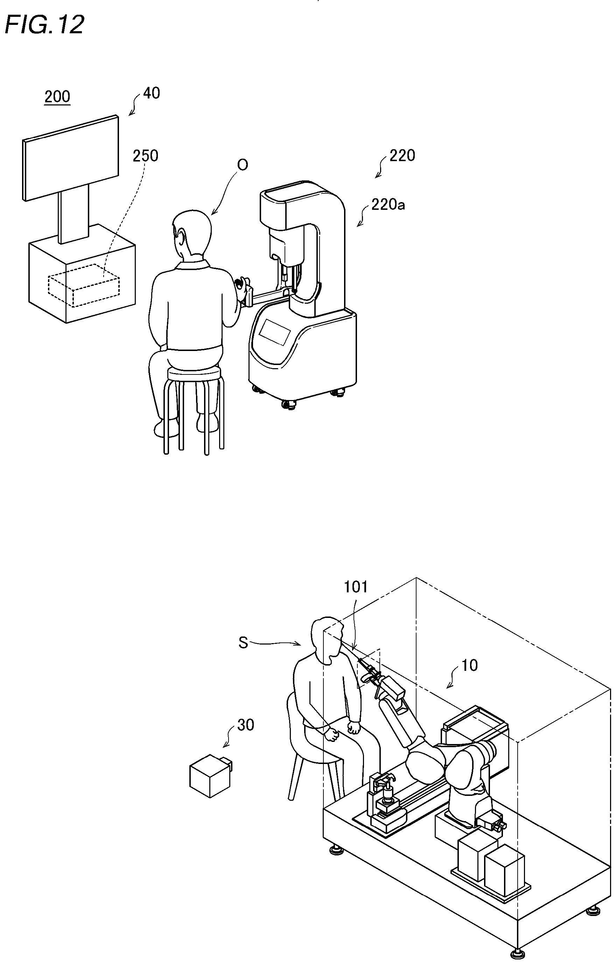

The configuration of a specimen collection robot system 200 according to a second embodiment is now described with reference to FIGS. 12 to 15. The specimen collection robot system 200 includes a first operator 220a and a second operator 220b in a master robot 220, unlike the first embodiment in which the first operator 20a and the second operator 20b are provided in the master robot 20. The same or similar configurations as those of the first embodiment are denoted by the same reference numerals, and detailed description thereof is omitted.

As shown in FIG. 12, the specimen collection robot system 200 according to the second embodiment includes a slave robot 10, the master robot 220, an imager 30, a display 40, and a controller 250. The specimen collection robot system 200 is an example of a "robot system" in the claims.

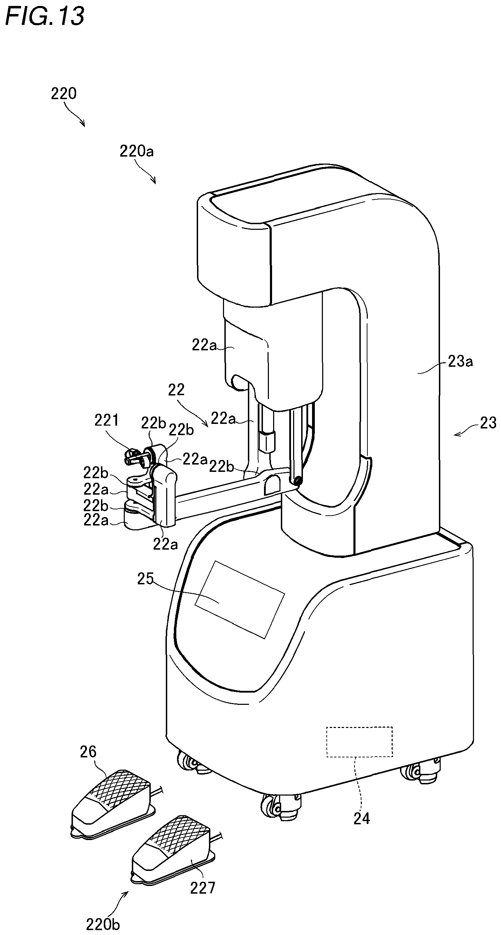

As shown in FIG. 13, the master robot 220 includes the first operator 220a and the second operator 220b. Straight-ahead movement of a hand 12 and rotational movement other than rotational movement of a specimen collection member 101 are assigned to the first operator 220a. Specifically, the straight-ahead movement of the hand 12 in a forward-rearward direction (A1 direction), a right-left direction (A2 direction), and an upward-downward direction (A3 direction) and the rotational movement of the hand 12 in a second rotation direction (C2 direction) and a third rotation direction (C3 direction) are assigned to the first operator 220a. The rotational movement of the specimen collection member 101 of the hand 12 is assigned to the second operator 220b. Specifically, the rotational movement of the hand 12 in a first rotation direction (C1 direction) is assigned to the second operator 20b. The first operator 220a is an example of a "first operator" in the claims. The second operator 220b is an example of a "second operator" in the claims.

The first operator 220a includes a movable operation handle 221, an arm 22, a gantry 23, a control unit 24, an operation panel 25, and a clutch pedal 26. The operation handle 221 is operated by one hand (the right hand in FIG. 12) of an operation person O. The operation handle 221 is provided to perform the straight-ahead movement of the hand 12 and the rotational movement of the hand 12 other than the rotational movement of the specimen collection member 101 of the hand 12. Specifically, the operation handle 221 is provided to move the hand 12 straight ahead in the forward-rearward direction (A1 direction), the right-left direction (A2 direction), and the upward-downward direction (A3 direction) and rotationally move the hand 12 in the second rotation direction (C2 direction) and the third rotation direction (C3 direction). The operation handle 221 is an example of a "third handle" in the claims.

The second operator 220b includes a foot pedal 227 (foot switch). The foot pedal 227 is operated by the foot of the operation person O. The foot pedal 227 is provided to rotationally move the specimen collection member 101 of the hand 12. Specifically, the foot pedal 227 is provided to rotationally move the hand 12 in the first rotation direction (C1 direction). The foot pedal 227 outputs an operation signal by being depressed by the foot of the operation person O. The foot pedal 227 is provided separately from and independently of the clutch pedal 26.

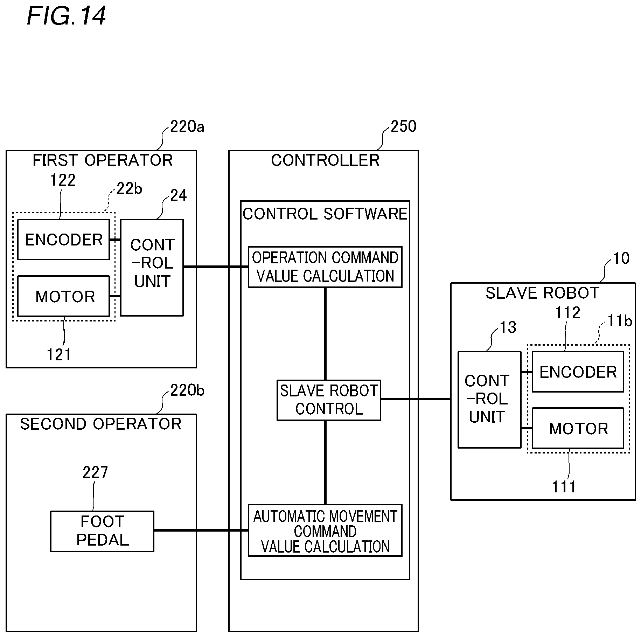

In the second embodiment, as shown in FIG. 14, the controller 250 controls the straight-ahead movement of the hand 12 and a portion of the rotational movement of the hand 12 independently of each other by dividing an operation system when a specimen is collected from a subject S by the specimen collection member 101 held by the hand 12. Specifically, when the specimen is collected from the subject S by the specimen collection member 101 held by the hand 12, the controller 250 controls the straight-ahead movement of the hand 12 and a portion of the rotational movement of the hand 12 independently of each other by dividing the operation system by a plurality of operators (the first operator 220a and the second operator 220b). Furthermore, the controller 250 controls the straight-ahead movement of the hand 12 and the rotational movement of the specimen collection member 101 of the hand 12 independently of each other by dividing the operation system.

In the second embodiment, the controller 250 controls at least the straight-ahead movement of the hand 12 and a portion of the rotational movement of the hand 12 independently of each other by controlling at least the straight-ahead movement of the hand 12 based on an operation on the first operator 220a by the operation person 0 and a portion of the rotational movement of the hand 12 based on an operation on the second operator 220b by the operation person 0 to divide the operation system. Specifically, the controller 250 controls the straight-ahead movement of the hand 12 and the movement of the hand 12 other than the rotational movement of the specimen collection member 101 of the hand 12 based on an operation on the operation handle 221 of the first operator 220a, and controls the rotational movement of the specimen collection member 101 of the hand 12 based on an operation on the foot pedal 227 of the second operator 220b. More specifically, the controller 250 controls the straight-ahead movement of the hand 12 in the forward-rearward direction (A1 direction), the right-left direction (A2 direction), and the upward-downward direction (A3 direction) and the rotational movement of the hand 12 in the second direction (C2 direction) and the third direction (C3 direction) based on an operation on the operation handle 221 of the first operator 220a, and controls the rotational movement of the hand 12 in the first direction (C1 direction) based on an operation on the foot pedal 227 of the second operator 220b.

When the operation handle 221 of the first operator 220a of the master robot 220 is operated by the operation person O, the control unit 24 of the first operator 220a of the master robot 220 outputs the operation amount of the operation handle 221 to the controller 250 based on the detection result of the encoder 122. The controller 250 calculates an operation command value for the slave robot 10 based on the operation amount of the operation handle 221 output from the control unit 24. When the operation handle 221 is not operated by the operation person O, the operation command value is zero.

When the foot pedal 227 of the second operator 220b of the master robot 220 is operated by the operation person O, the foot pedal 227 of the second operator 220b of the master robot 220 outputs an operation signal to the controller 250. The controller 250 calculates an automatic movement command value for the slave robot 10 (a command value for rotation of the specimen collection member 101) based on the operation signal from the foot pedal 227. When the foot pedal 227 is not operated by the operation person O, the automatic movement command value is zero.

The controller 250 then calculates a movement command value for the slave robot 10 based on the calculated operation command value and automatic movement command value, and outputs the calculated movement command value to a control unit 13 of the slave robot 10. The control unit 13 of the slave robot 10 controls the operation of motors 111 of joints 11b based on the movement command value output from the controller 250. Consequently, the movement of the hand 12 of the slave robot 10 is controlled so as to correspond to operations on the operation handle 221 and the foot pedal 227 of the master robot 220.

### Control Process for Specimen Collection Work

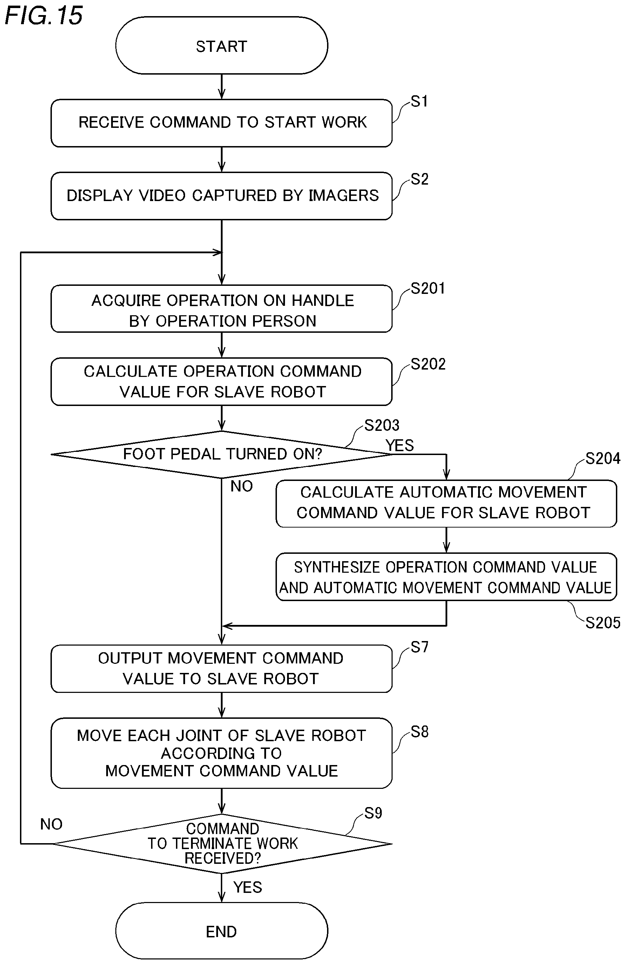

A control process for specimen collection work of the specimen collection robot system 200 according to the second embodiment is now described based on a flowchart with reference to FIG. 15. The same or similar process operations as those of the first embodiment are denoted by the same reference numerals, and detailed description thereof is omitted.

As shown in FIG. 15, in step S201, the operation amount of the operation handle 221 of the first operator 220a by the operation person 0 is acquired.

Then, in step S202, the operation command value for the slave robot 10 is calculated based on the operation amount of the operation handle 221 by the operation person 0 acquired in step S201.

Then, in step S203, it is determined whether or not the foot pedal 227 of the second operator 220b is turned on (operated) by the operation person O. When it is determined that the foot pedal 227 is turned on based on the output of the operation signal from the foot pedal 227, the process advances to step S204.

Then, in step S204, the automatic movement command value for the slave robot 10 is calculated based on an operation on the foot pedal 227.

Then, in step S205, the operation command value calculated in step S202 and the automatic movement command value calculated in step S204 are synthesized. Thus, the movement command value for the slave robot 10 is calculated. Then, the process advances to step S7.

When it is determined in step S203 that the foot pedal 227 is not turned on based on no output of the operation signal from the foot pedal 227, the process advances to step S7 without performing the process operations in step S204 and step S205.

Then, the movement command value is output to the slave robot 10 in step S7.

Then, in step S8, the movement of each joint 11b of the slave robot 10 is controlled based on the movement command value output in step S7.

The remaining configurations of the second embodiment are similar to those of the first embodiment.

### Advantages of Second Embodiment

According to the second embodiment, the following advantages are achieved.

According to the second embodiment, as described above, the specimen collection robot system 200 includes the controller 250 configured or programmed to control the straight-ahead movement of the hand 12 and at least a portion of the rotational movement of the hand 12 independently of each other by dividing the operation system when a specimen is collected from the subject S by the specimen collection member 101 held by the hand 12. Accordingly, it is possible to improve the operability when the hand 12 of the slave robot 10 is remotely controlled by the master robot 220 to perform a procedure on the subject S (collect a specimen from the subject S), similarly to the first embodiment.

According to the second embodiment, as described above, the first operator 220a includes the operation handle 221 operated by one hand of the operation person O to perform the straight-ahead movement of the hand 12 and the rotational movement of the hand 12 other than the rotational movement of the specimen collection member 101 of the hand 12, and the second operator 220b includes the foot pedal 227 operated by the foot of the operation person 0 to perform the rotational movement of the specimen collection member 101 of the hand 12. The controller 250 is configured or programmed to control the straight-ahead movement of the hand 12 and the rotational movement of the hand 12 other than the rotational movement of the specimen collection member 101 of the hand 12 based on an operation on the operation handle 221, and controls the rotational movement of the specimen collection member 101 of the hand 12 based on an operation on the foot pedal 227. Accordingly, an operation for the straight-ahead movement of the hand 12 and an operation for the rotational movement of the hand 12 other than the rotational movement of the specimen collection member 101 of the hand 12 can be performed by one hand of the operation person O, and an operation for the rotational movement of the specimen collection member 101 of the hand 12 can be performed by the foot of the operation person O. Consequently, erroneous operations for the rotational movement of the specimen collection member 101 of the hand 12 and another movement (the straight-ahead movement of the hand 12 and the rotational movement of the hand 12 other than the rotational movement of the specimen collection member 101 of the hand 12) can be easily reduced or prevented. Thus, unintended and erroneous movement of the hand 12 caused by an erroneous operation can be easily reduced or prevented.

The remaining advantages of the second embodiment are similar to those of the first embodiment.

### Third Embodiment

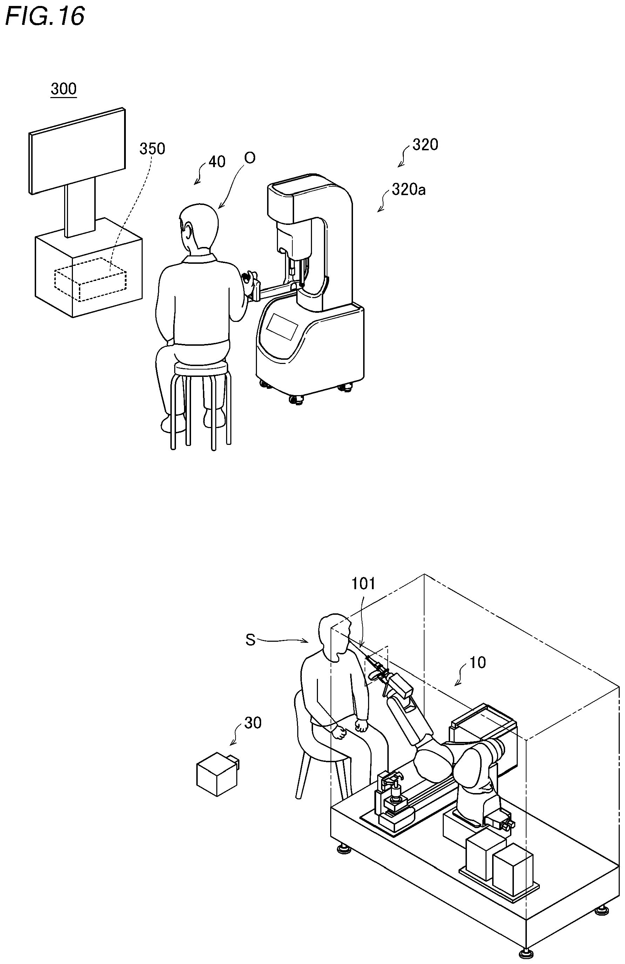

The configuration of a specimen collection robot system 300 according to a third embodiment is now described with reference to FIGS. 16 to 19. The specimen collection robot system 300 automatically rotationally moves a specimen collection member 101 of a hand 12 regardless of an operation of an operation person 0, unlike the second embodiment in which the specimen collection member 101 of the hand 12 is rotationally moved by an operation on the foot pedal 227 by the operation person O. The same or similar configurations as those of the first or second embodiment are denoted by the same reference numerals, and detailed description thereof is omitted.

As shown in FIG. 16, the specimen collection robot system 300 according to the third embodiment includes a slave robot 10, a master robot 320, an imager 30, a display 40, and a controller 350. The specimen collection robot system 300 is an example of a "robot system" in the claims.

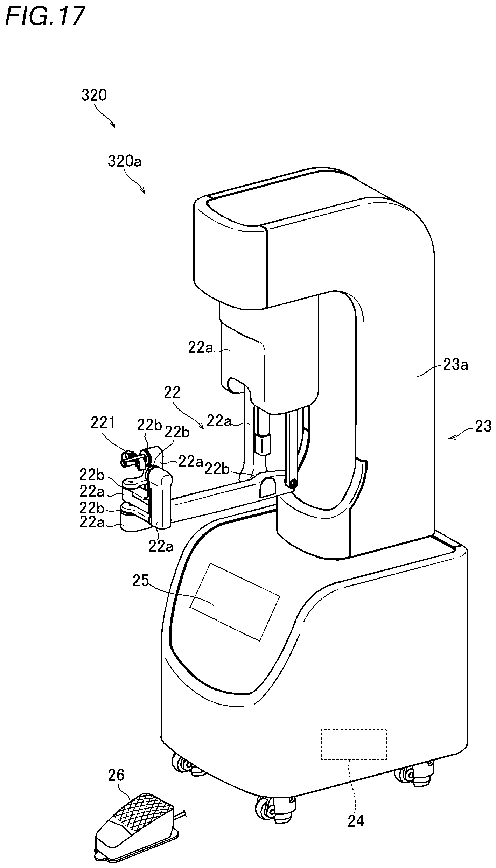

As shown in FIG. 17, the master robot 320 includes an operator 320a having substantially the same configuration as the first operator 220a according to the second embodiment, and does not include the second operator 220b according to the second embodiment. The master robot 320 does not include the foot pedal 227 according to the second embodiment. The operator 320a includes an operation handle 221, an arm 22, a gantry 23, a control unit 24, an operation panel 25, and a clutch pedal 26.

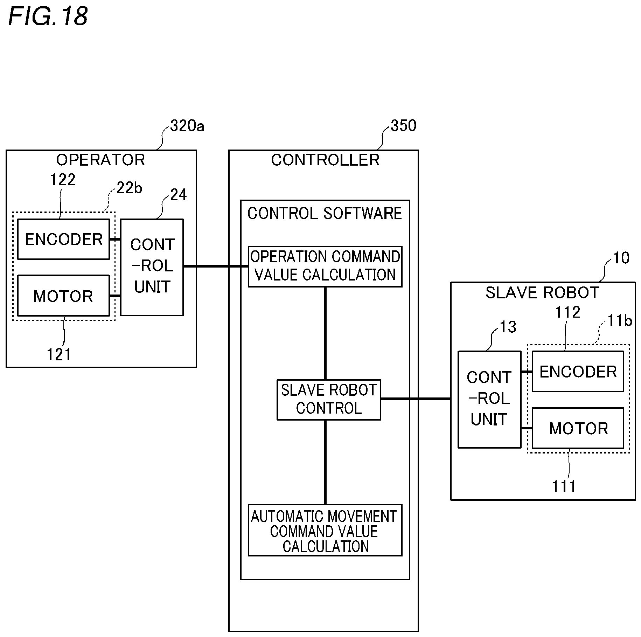

As shown in FIG. 18, in the third embodiment, the controller 350 controls at least straight-ahead movement of the hand 12 and a portion of rotational movement of the hand 12 independently of each other by dividing an operation system when a specimen is collected from a subject S by the specimen collection member 101 held by the hand 12. Specifically, when the specimen is collected from the subject S by the specimen collection member 101 held by the hand 12, the controller 350 controls at least the straight-ahead movement of the hand 12 and a portion of the rotational movement of the hand 12 independently of each other by automating the rotational movement to divide the operation system. More specifically, the controller 350 controls at least the straight-ahead movement of the hand 12 and the rotational movement of the specimen collection member 101 of the hand 12 independently of each other by automatically rotationally moving the specimen collection member 101 of the hand 12 without an operation for the rotational movement of the specimen collection member 101 of the hand 12 by the operation person 0 to divide the operation system.

In the third embodiment, the controller 350 always automatically rotationally moves the specimen collection member 101 of the hand 12 without an operation for the rotational movement of the specimen collection member 101 of the hand 12 by the operation person O. Specifically, the controller 350 always automatically rotationally moves the specimen collection member 101 of the hand 12 without an operation for the rotational movement of the specimen collection member 101 of the hand 12 by the operation person 0 regardless of the insertion state of the specimen collection member 101 into the nasal cavity of the subject S during specimen collection work. That is, the controller 350 automatically starts to rotationally move the specimen collection member 101 of the hand 12 without an operation for the rotational movement of the specimen collection member 101 of the hand 12 by the operation person 0 before the specimen collection member 101 is inserted into the nasal cavity of the subject S.

In the third embodiment, the controller 350 controls the straight-ahead movement of the hand 12 and the movement of the hand 12 other than the rotational movement of the specimen collection member 101 of the hand 12 based on an operation on the operation handle 221 of the operator 320a, and automatically controls the rotational movement of the specimen collection member 101 of the hand 12 regardless of an operation of the operation person O. Specifically, the controller 350 controls the straight-ahead movement of the hand 12 in a forward-rearward direction (A1 direction), a right-left direction (A2 direction), and an upward-downward direction (A3 direction) and the rotational movement of the hand 12 in a second rotation direction (C2 direction) and a third rotation direction (C3 direction) based on an operation on the operation handle 221 of the operator 320a, and controls the rotational movement of the hand 12 in a first rotation direction (C1 direction) regardless of an operation of the operation person O.

When the operation handle 221 of the operator 320a of the master robot 320 is operated by the operation person O, the control unit 24 of the operator 320a of the master robot 320 outputs the operation amount of the operation handle 221 to the controller 350 based on the detection result of an encoder 122. Then, the controller 350 calculates an operation command value for the slave robot 10 based on the operation amount of the operation handle 221 output from the control unit 24. When the operation handle 221 is not operated by the operation person O, the operation command value is zero.

The controller 350 calculates an automatic movement command value for the slave robot 10 (a command value for rotation of the specimen collection member 101) regardless of an operation of the operation person O.

Then, the controller 350 calculates a movement command value for the slave robot 10 based on the calculated operation command value and automatic movement command value, and outputs the calculated movement command value to a control unit 13 of the slave robot 10. Then, the control unit 13 of the slave robot 10 controls the operation of motors 111 of joints 11b based on the movement command value output from the controller 350. Consequently, the movement of the hand 12 of the slave robot 10 is controlled so as to correspond to the operation handle 221 of the master robot 320 and the automatic rotation of the specimen collection member 101.

### Control Process for Specimen Collection Work

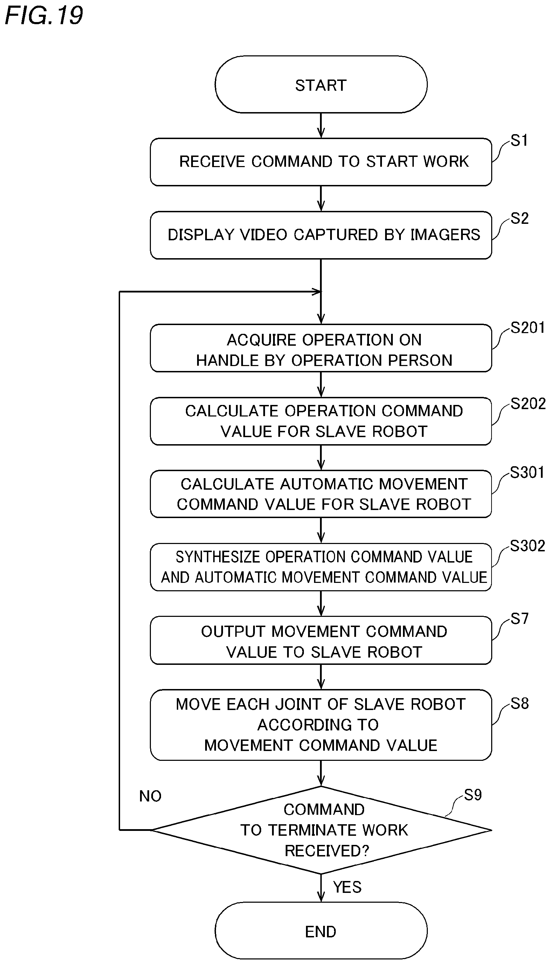

A control process for specimen collection work of the specimen collection robot system 300 according to the third embodiment is now described based on a flowchart with reference to FIG. 19. The same or similar process operations as those of the first or second embodiment are denoted by the same reference numerals, and detailed description thereof is omitted.

As shown in FIG. 19, in step S301, the automatic movement command value for the slave robot 10 is calculated regardless of an operation of the operation person 0.

Then, in step S302, the operation command value calculated in step S202 and the automatic movement command value calculated in step S301 are synthesized. Thus, the movement command value for the slave robot 10 is calculated.

Then, the movement command value is output to the slave robot 10 in step S7.

Then, in step S8, the movement of each joint 11b of the slave robot 10 is controlled based on the movement command value output in step S7.

The remaining configurations of the third embodiment are similar to those of the first or second embodiment.

### Advantages of Third Embodiment

According to the third embodiment, the following advantages are achieved.

According to the third embodiment, as described above, the specimen collection robot system 300 includes the controller 350 configured or programmed to control the straight-ahead movement of the hand 12 and at least a portion of the rotational movement of the hand 12 independently of each other by dividing the operation system when a specimen is collected from the subject S by the specimen collection member 101 held by the hand 12. Accordingly, it is possible to improve the operability when the hand 12 of the slave robot 10 is remotely controlled by the master robot 220 to perform a procedure on the subject S (collect a specimen from the subject S), similarly to the first and second embodiments.

According to the third embodiment, as described above, the controller 350 is configured or programmed to control the straight-ahead movement of the hand 12 and the rotational movement of the specimen collection member 101 of the hand 12 independently of each other by automatically rotationally moving the specimen collection member 101 of the hand 12 without an operation for the rotational movement of the specimen collection member 101 of the hand 12 by the operation person O to divide the operation system. Accordingly, the operation person O does not need to perform an operation to rotationally move the specimen collection member 101 of the hand 12, and thus erroneous operations for the straight-ahead movement of the hand 12 and the rotational movement of the specimen collection member 101 of the hand 12 can be easily reduced or prevented. Consequently, unintended and erroneous movement of the hand 12 caused by an erroneous operation can be easily reduced or prevented.

According to the third embodiment, as described above, the controller 350 is configured or programmed to always automatically rotationally move the specimen collection member 101 of the hand 12 without an operation for the rotational movement of the specimen collection member 101 of the hand 12 by the operation person O. Accordingly, the specimen collection member 101 of the hand 12 is always automatically rotationally moved without an operation for the rotational movement of the specimen collection member 101 of the hand 12 by the operation person O, and thus the specimen collection member 101 of the hand 12 can always be reliably rotationally moved. Consequently, when the specimen collection member 101 reaches a specimen collection position of the subject, a specimen can be reliably scraped at the specimen collection position of the subject by the specimen collection member 101.

The remaining advantages of the third embodiment are similar to those of the first or second embodiment.

### Fourth Embodiment

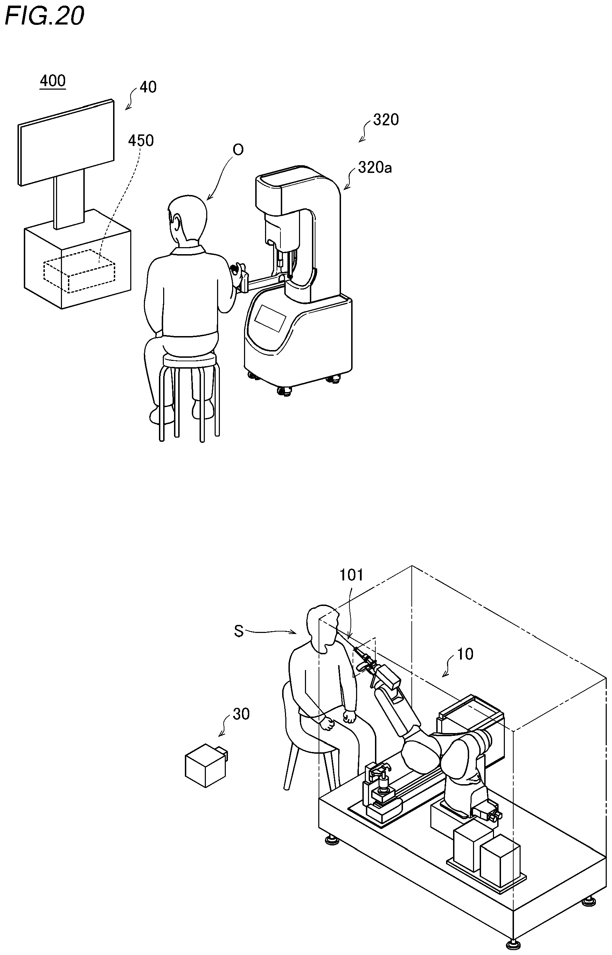

The configuration of a specimen collection robot system 400 according to a fourth embodiment is now described with reference to FIGS. 20 to 22. The specimen collection robot system 400 automatically rotationally moves a specimen collection member 101 of a hand 12 after the specimen collection member 101 is inserted into a subject S to a predetermined depth, unlike the third embodiment in which the specimen collection member 101 of the hand 12 is always automatically rotationally moved. The same or similar configurations as those of the first, second, or third embodiment are denoted by the same reference numerals, and detailed description thereof is omitted.

As shown in FIG. 20, the specimen collection robot system 400 according to the fourth embodiment includes a slave robot 10, a master robot 320, an imager 30, a display 40, and a controller 450. The specimen collection robot system 400 is an example of a "robot system" in the claims.

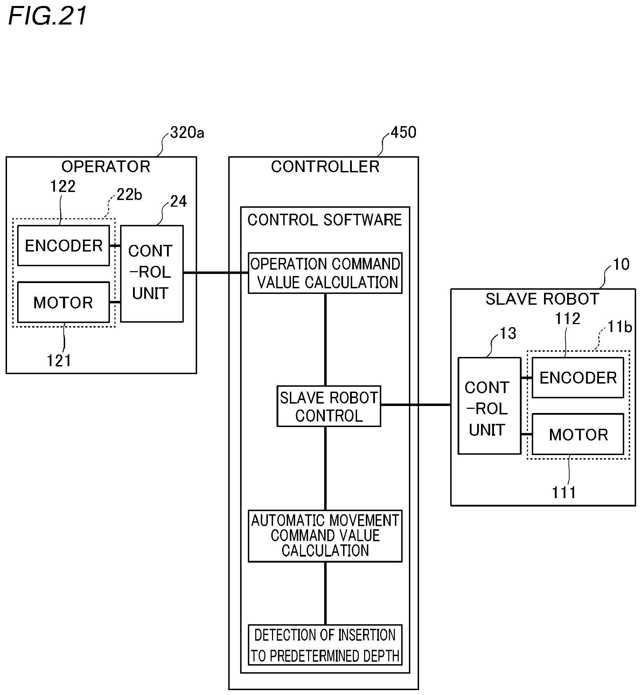

As shown in FIG. 21, in the fourth embodiment, the controller 450 controls at least straight-ahead movement of the hand 12 and a portion of rotational movement of the hand 12 independently of each other by dividing an operation system when a specimen is collected from the subject S by the specimen collection member 101 held by the hand 12. Specifically, when the specimen is collected from the subject S by the specimen collection member 101 held by the hand 12, the controller 450 controls at least the straight-ahead movement of the hand 12 and a portion of the rotational movement of the hand 12 independently of each other by automating the rotational movement to divide the operation system. More specifically, the controller 450 controls at least the straight-ahead movement of the hand 12 and the rotational movement of the specimen collection member 101 of the hand 12 independently of each other by automatically rotationally moving the specimen collection member 101 of the hand 12 without an operation for the rotational movement of the specimen collection member 101 of the hand 12 by an operation person O to divide the operation system.

In the fourth embodiment, the controller 450 automatically rotationally moves the specimen collection member 101 of the hand 12 based on the specimen collection member 101 being inserted into the subject S to the predetermined depth without an operation for the rotational movement of the specimen collection member 101 of the hand 12 by the operation person O. Specifically, the controller 450 does not rotationally move the specimen collection member 101 of the hand 12 before the specimen collection member 101 is inserted into the nasal cavity of the subject S during specimen collection work. After the specimen collection member 101 is inserted into the nasal cavity of the subject S to the predetermined depth, the controller 450 automatically rotationally moves the specimen collection member 101 of the hand 12 without an operation for the rotational movement of the specimen collection member 101 of the hand 12 by the operation person O.

The controller 450 detects that the specimen collection member 101 has been inserted into the subject S to the predetermined depth based on at least one of an image (side image) of the subject S and the specimen collection member 101 captured by the imager 30 or position information (coordinate information) on the slave robot 10. The controller 450 detects that the specimen collection member 101 has been inserted into the subject S to the predetermined depth by detecting the insertion state of the specimen collection member 101 into the nasal cavity of the subject S based on the recognition result of the image of the subject S and the specimen collection member 101 captured by the imager 30 and the position information on the hand 12 of the slave robot 10. Calculation of a movement command value for the slave robot 10 by the controller 450 is the same as that in the third embodiment, except that an automatic movement command value is calculated based on the specimen collection member 101 being inserted into the subject S to the predetermined depth.

### Control Process for Specimen Collection Work

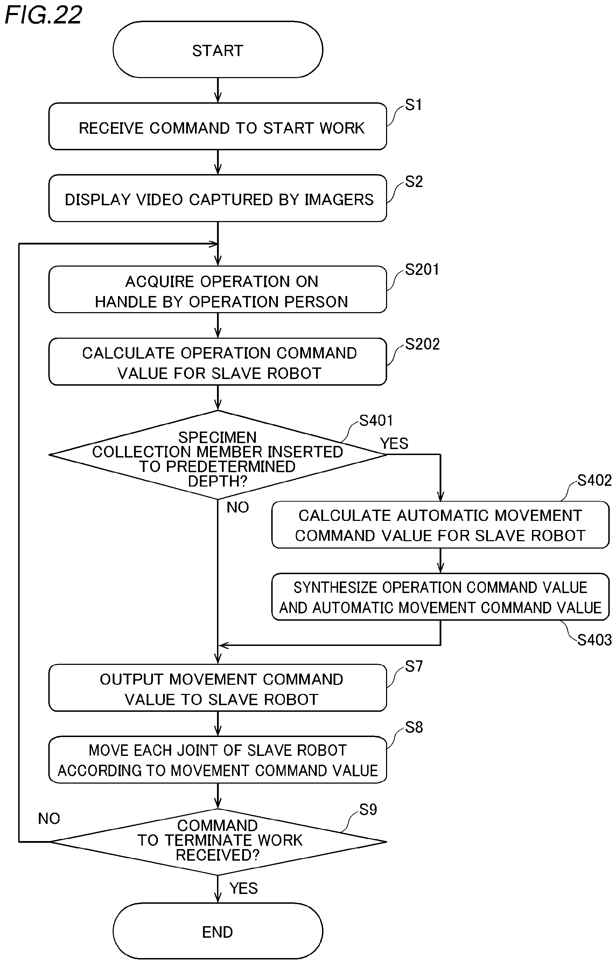

A control process for specimen collection work of the specimen collection robot system 400 according to the fourth embodiment is now described based on a flowchart with reference to FIG. 22. The same or similar process operations as those of the first, second, or third embodiment are denoted by the same reference numerals, and detailed description thereof is omitted.

As shown in FIG. 22, in step S401, it is determined whether or not the specimen collection member 101 has been inserted into the subject S to the predetermined depth based on at least one of the image of the subject S and the specimen collection member 101 captured by the imager 30 or the position information on the slave robot 10. When it is determined that the specimen collection member 101 has been inserted into the subject S to the predetermined depth, the process advances to step S402.

Then, in step S402, the automatic movement command value for the slave robot 10 is calculated regardless of an operation of the operation person O.

Then, in step S403, an operation command value calculated in step S202 and the automatic movement command value calculated in step S402 are synthesized. Thus, the movement command value for the slave robot 10 is calculated. Then, the process advances to step S7.

When it is determined in step S401 that the specimen collection member 101 has not been inserted into the subject S to the predetermined depth, the process advances to step S7 without performing the process operations in step S402 and step S403.

Then, the movement command value is output to the slave robot 10 in step S7.

Then, in step S8, movement of each joint 11b of the slave robot 10 is controlled based on the movement command value output in step S7.

The remaining configurations of the fourth embodiment are similar to those of the first, second, or third embodiment.

### Advantages of Fourth Embodiment

According to the fourth embodiment, the following advantages are achieved.

According to the fourth embodiment, as described above, the specimen collection robot system 400 includes the controller 450 configured or programmed to control the straight-ahead movement of the hand 12 and at least a portion of the rotational movement of the hand 12 independently of each other by dividing the operation system when a specimen is collected from the subject S by the specimen collection member 101 held by the hand 12. Accordingly, it is possible to improve the operability when the hand 12 of the slave robot 10 is remotely controlled by the master robot 220 to perform a procedure on the subject S (collect a specimen from the subject S), similarly to the first to third embodiments.

According to the fourth embodiment, as described above, the controller 450 is configured or programmed to automatically rotationally move the specimen collection member 101 of the hand 12 based on the specimen collection member 101 being inserted into the subject S to the predetermined depth without an operation for the rotational movement of the specimen collection member 101 of the hand 12 by the operation person O. Accordingly, the specimen collection member 101 of the hand 12 is not rotationally moved before the specimen collection member 101 is inserted into the subject S to the predetermined depth, and thus displacement of the axis of the specimen collection member 101 due to rotation of the specimen collection member 101 before insertion of the specimen collection member 101 into the subject S can be reduced or prevented. Consequently, the specimen collection member 101 can be easily inserted into the subject S. After the specimen collection member 101 is inserted into the subject S to the predetermined depth, the specimen collection member 101 of the hand 12 is rotationally moved, and thus a specimen can be reliably scraped at a specimen collection position of the subject S by the specimen collection member 101.

The remaining advantages of the fourth embodiment are similar to those of the first, second, or third embodiment.

### Fifth Embodiment

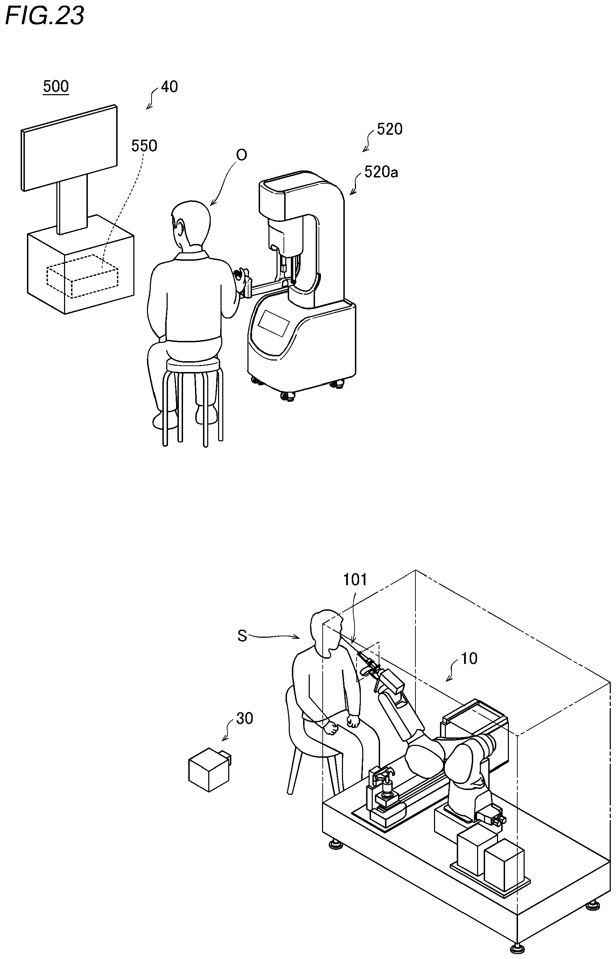

The configuration of a specimen collection robot system 500 according to a fifth embodiment is now described with reference to FIGS. 23 to 27. The specimen collection robot system 500 includes switches 521a on an operation handle 521 of an operator 520a of a master robot 520 to restrict movement of a hand 12 other than rotational movement of a specimen collection member 101 of the hand 12, unlike the first to fourth embodiments. The same or similar configurations as those of the first, second, third, or fourth embodiment are denoted by the same reference numerals, and detailed description thereof is omitted.

As shown in FIG. 23, the specimen collection robot system 500 according to the fifth embodiment includes a slave robot 10, the master robot 520, an imager 30, a display 40, and a controller 550. The specimen collection robot system 500 is an example of a "robot system" in the claims.

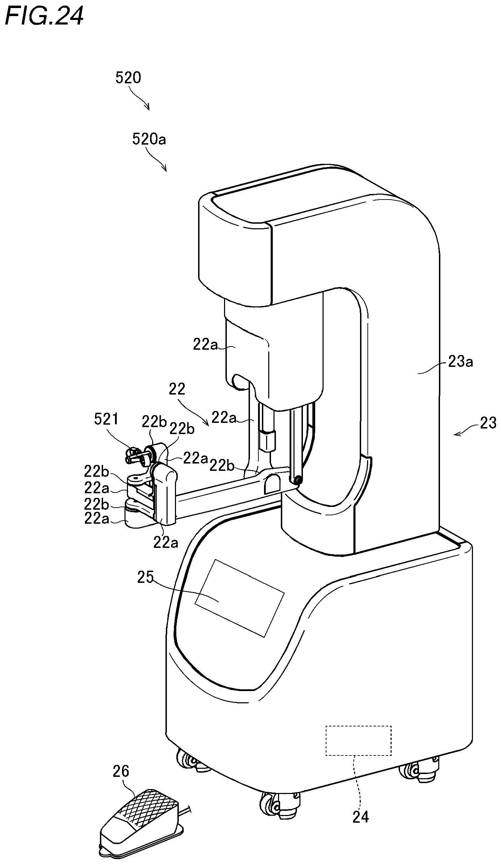

As shown in FIG. 24, the master robot 520 includes the operator 520a. Straight-ahead movement of the hand 12 and rotational movement of the hand 12 are assigned to the operator 520a. Specifically, the straight-ahead movement of the hand 12 in a forward-rearward direction (A1 direction), a right-left direction (A2 direction), and an upward-downward direction (A3 direction) and the rotational movement of the hand 12 in a first rotation direction (C1 direction), a second rotation direction (C2 direction), and a third rotation direction (C3 direction) are assigned to the operator 520a. The operator 520a is an example of a "third operator" in the claims.

The operator 520a includes the movable operation handle 521, an arm 22, a gantry 23, a control unit 24, an operation panel 25, and a clutch pedal 26. The operation handle 521 is operated by one hand (the right hand in FIG. 23) of an operation person O. The operation handle 521 is provided to move the hand 12 straight ahead and move the hand 12 rotationally. Specifically, the operation handle 521 is provided to move the hand 12 straight ahead in the forward-rearward direction (A1 direction), the right-left direction (A2 direction), and the upward-downward direction (A3 direction) and rotationally move the hand 12 in the first rotation direction (C1 direction), the second rotation direction (C2 direction), and the third rotation direction (C3 direction). The operation handle 521 is an example of a "fourth handle" in the claims.

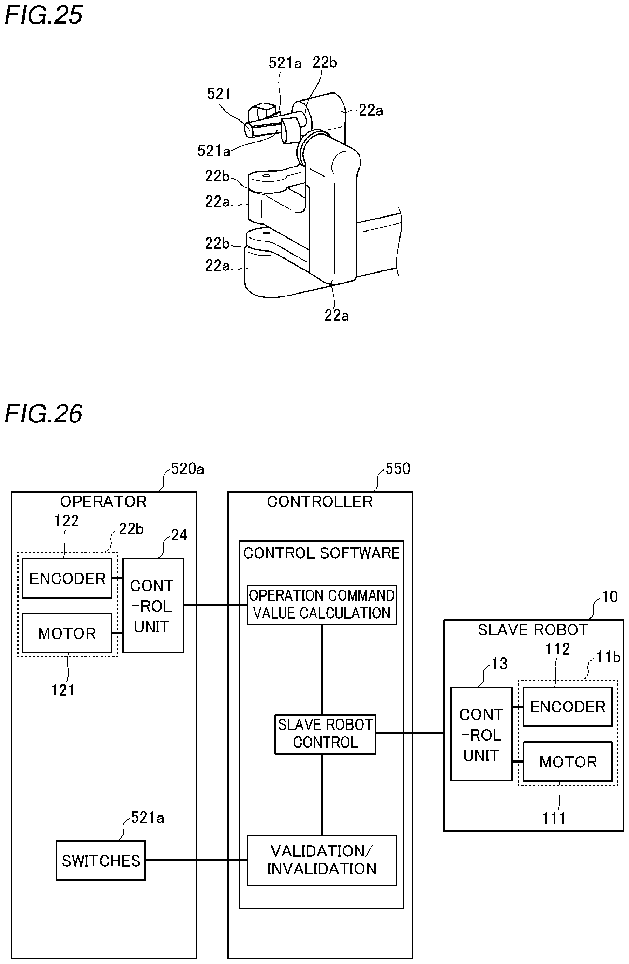

As shown in FIG. 25, in the fifth embodiment, the switches 521a (grip switches) are provided on the operation handle 521. The switches 521a are provided to restrict the movement of the hand 12 other than the rotational movement of the specimen collection member 101 of the hand 12, and to enable the rotational movement of the specimen collection member 101 of the hand 12. A pair of switches 521a are provided, and are gripped and operated by the fingers of the operation person O. The switches 521a are provided on one side and another side of the operation handle 521 so as to sandwich the operation handle 521 therebetween.

As shown in FIG. 26, in the fifth embodiment, the controller 550 controls at least the straight-ahead movement of the hand 12 and a portion of the rotational movement of the hand 12 independently of each other by dividing an operation system when a specimen is collected from a subject S by the specimen collection member 101 held by the hand 12. Specifically, the controller 550 controls at least the straight-ahead movement of the hand 12 and a portion of the rotational movement of the hand 12 independently of each other by dividing the operation system by the switches 521a when a specimen is collected from the subject S by the specimen collection member 101 held by the hand 12. More specifically, the controller 550 controls at least the straight-ahead movement of the hand 12 and the rotational movement of the specimen collection member 101 of the hand 12 independently of each other by dividing the operation system by the switches 521a.

In the fifth embodiment, the controller 550 controls at least the straight-ahead movement of the hand 12 and the rotational movement of the specimen collection member 101 of the hand 12 independently of each other by performing a control based on an operation on the operation handle 521 of the operator 520a and an operation on the switches 521a to divide the operation system. Specifically, the controller 550 validates an operation command value for the straight-ahead movement of the hand 12 in the forward-rearward direction (A1 direction), an operation command value for the straight-ahead movement of the hand 12 in the right-left direction (A2 direction), an operation command value for the straight-ahead movement of the hand 12 in the upward-downward direction (A3 direction), an operation command value for the rotational movement of the hand 12 in the first rotation direction (C1 direction), an operation command value for the hand 12 in the second rotation direction (C2 direction), and an operation command value for the rotational movement of the hand 12 in the third rotation direction (C3 direction) when the operation handle 521 is operated while the switches 521a are turned on, and controls the movement of the hand 12.

The controller 550 invalidates the operation command values (the operation command value for the straight-ahead movement of the hand 12 in the forward-rearward direction (A1 direction), the operation command value for the straight-ahead movement of the hand 12 in the right-left direction (A2 direction), the operation command value for the straight-ahead movement of the hand 12 in the upward-downward direction (A3 direction), the operation command value for the hand 12 in the second rotation direction (C2 direction), and the operation command value for the rotational movement of the hand 12 in the third rotation direction (C3 direction)) other than the operation command value for the rotational movement of the hand 12 in the first rotation direction (C1 direction) and validates only the operation command value for the rotational movement of the hand 12 in the first rotation direction (C1 direction) when the operation handle 521 is operated while the switches 521a are turned off, and controls the movement of the hand 12. The controller 550 controls only the rotational movement of the hand 12 in the first rotation direction (C1 direction) based on an operation on the operation handle 521 when the switches 521a are turned off.

When the operation handle 521 of the operator 520a of the master robot 520 is operated by the operation person O, the control unit 24 of the operator 520a of the master robot 520 outputs the operation amount of the operation handle 521 to the controller 550 based on the detection result of an encoder 122. The controller 550 calculates the operation command value for the slave robot 10 based on the operation amount of the operation handle 521 output from the control unit 24. When the operation handle 521 is not operated by the operation person O, the operation command value is zero.

When the switches 521a are turned off, the controller 550 invalidates the operation command values (the operation command value for the straight-ahead movement of the hand 12 in the forward-rearward direction (A1 direction), the operation command value for the straight-ahead movement of the hand 12 in the right-left direction (A2 direction), the operation command value for the straight-ahead movement of the hand 12 in the upward-downward direction (A3 direction), the operation command value for the hand 12 in the second rotation direction (C2 direction), and the operation command value for the rotational movement of the hand 12 in the third rotation direction (C3 direction)) other than the operation command value for the rotational movement of the hand 12 in the first rotation direction (C1 direction), validates only the operation command value for the rotational movement of the hand 12 in the first rotation direction (C1 direction), and calculates a movement command value for the slave robot 10.

When the switches 521a are turned on, the controller 550 validates the operation command value for the straight-ahead movement of the hand 12 in the forward-rearward direction (A1 direction), the operation command value for the straight-ahead movement of the hand 12 in the right-left direction (A2 direction), the operation command value for the straight-ahead movement of the hand 12 in the upward-downward direction (A3 direction), the operation command value for the rotational movement of the hand 12 in the first rotation direction (C1 direction), the operation command value for the hand 12 in the second rotation direction (C2 direction), and the operation command value for the rotational movement of the hand 12 in the third rotation direction (C3 direction), and calculates the movement command value for the slave robot 10.

Then, the controller 550 outputs the calculated movement command value to the control unit 13 of the slave robot 10. Then, the control unit 13 of the slave robot 10 controls the operation of motors 111 of joints 11b based on the movement command value output from the controller 550. Consequently, the movement of the hand 12 of the slave robot 10 is controlled so as to correspond to operations on the operation handle 521 and the switches 521a of the master robot 520.

### Control Process for Specimen Collection Work

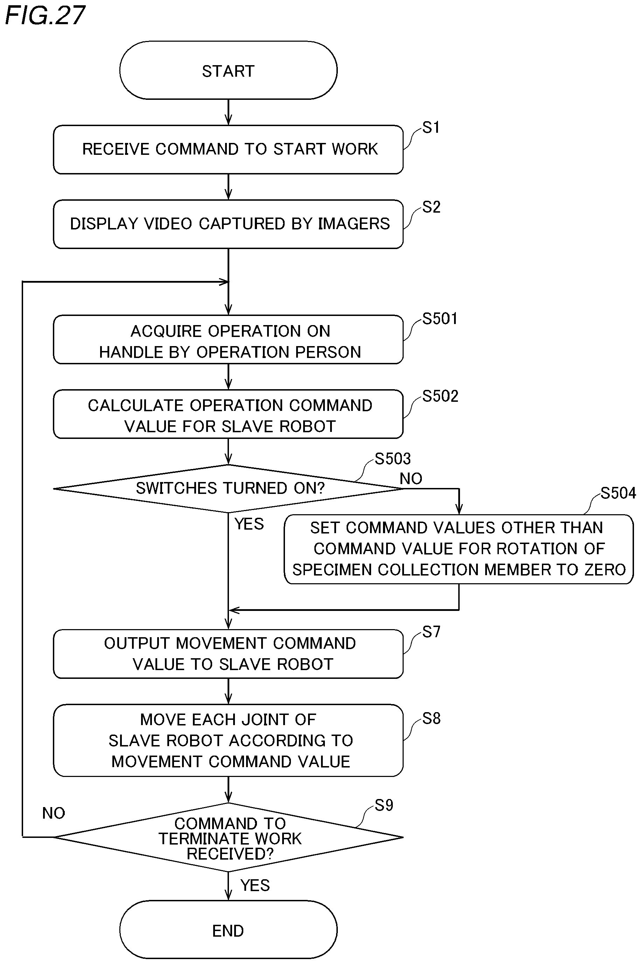

A control process for specimen collection work of the specimen collection robot system 500 according to the fifth embodiment is now described based on a flowchart with reference to FIG. 27. The same or similar process operations as those of the first embodiment are denoted by the same reference numerals, and detailed description thereof is omitted.

As shown in FIG. 27, in step S501, the operation amount of the operation handle 521 by the operation person O is acquired.

Then, in step S502, the operation command value for the slave robot 10 is calculated based on the operation amount of the operation handle 521 acquired in step S501.

Then, in step S503, it is determined whether or not the switches 521a are turned on. When it is determined that the switches 521a are not turned on, the process advances to step S504.

Then, in step S504, the operation command values other than the operation command value for the rotational movement of the specimen collection member 101 of the hand 12 are set to zero. In step S504, the operation command values (the operation command value for the straight-ahead movement of the hand 12 in the forward-rearward direction, the operation command value for the straight-ahead movement of the hand 12 in the right-left direction, the operation command value for the straight-ahead movement of the hand 12 in the upward-downward direction, the operation command value for the hand 12 in the second rotation direction, and the operation command value for the rotational movement of the hand 12 in the third rotation direction) other than the operation command value for the rotational movement of the hand 12 in the first rotation direction are invalidated. Then, the process advances to step S7.

When it is determined in step S503 that the switches 521a are turned on, the process advances to step S7 without performing the process operation in step S504.

Then, the movement command value is output to the slave robot 10 in step S7.

Then, in step S8, the movement of each joint 11b of the slave robot 10 is controlled based on the movement command value output in step S7.

The remaining configurations of the fifth embodiment are similar to those of the first, second, third, or fourth embodiment.

### Advantages of Fifth Embodiment

According to the fifth embodiment, the following advantages are achieved.

According to the fifth embodiment, as described above, the specimen collection robot system 500 includes the controller 450 configured or programmed to control the straight-ahead movement of the hand 12 and at least a portion of the rotational movement of the hand 12 independently of each other by dividing the operation system when a specimen is collected from the subject S by the specimen collection member 101 held by the hand 12. Accordingly, it is possible to improve the operability when the hand 12 of the slave robot 10 is remotely controlled by the master robot 520 to perform a procedure on the subject S (collect a specimen from the subject S), similarly to the first to fourth embodiments.

According to the fifth embodiment, as described above, the master robot 520 includes the operator 520a to which the straight-ahead movement and the rotational movement of the hand 12 are assigned. The operator 520a includes the operation handle 521 operated by one hand of the operation person O. The operation handle 521 includes the switches 521a to restrict the movement of the hand 12 other than the rotational movement of the specimen collection member 101 of the hand 12 and enable the rotational movement of the specimen collection member 101 of the hand 12. Furthermore, the controller 550 is configured or programmed to control the straight-ahead movement of the hand 12 and the rotational movement of the specimen collection member 101 of the hand 12 independently of each other by performing a control based on an operation on the operation handle 521 and an operation on the switches 521a to divide the operation system. Accordingly, restriction on the movement of the hand 12 other than the rotational movement of the specimen collection member 101 of the hand 12 can be switched by operating the switches 521a, and thus erroneous operations for the rotational movement of the specimen collection member 101 of the hand 12 and the movement of the hand 12 other than the rotational movement of the specimen collection member 101 can be easily reduced or prevented. Consequently, unintended and erroneous movement of the hand 12 caused by an erroneous operation can be easily reduced or prevented.

The remaining advantages of the fifth embodiment are similar to those of the first, second, third, or fourth embodiment.

### Sixth Embodiment

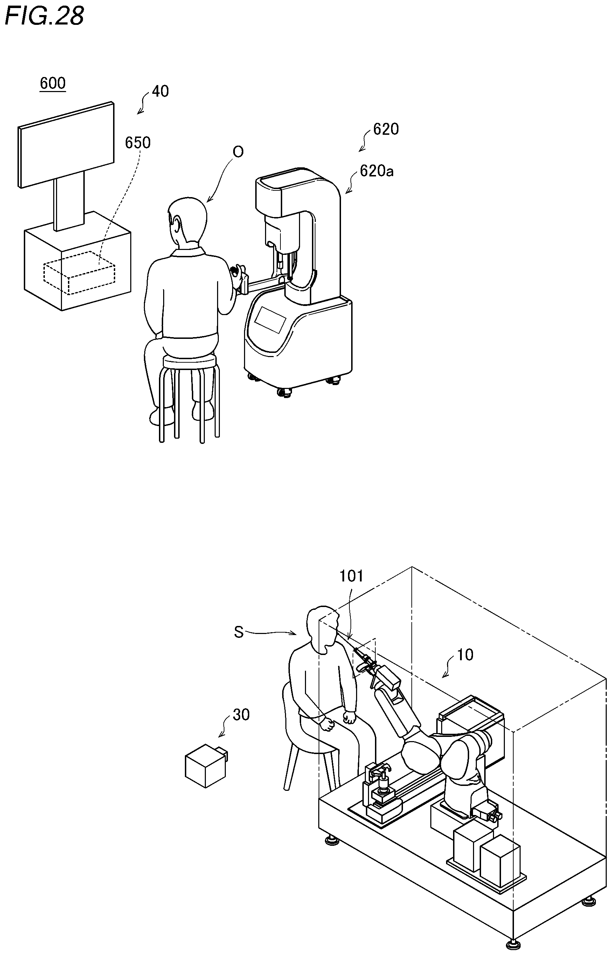

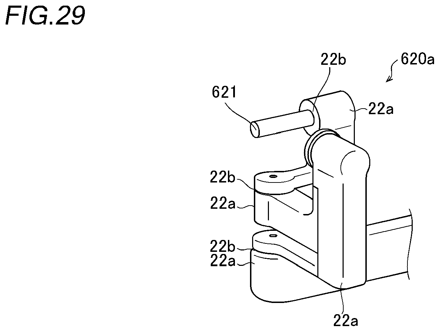

The configuration of a specimen collection robot system 600 according to a sixth embodiment is now described with reference to FIGS. 28 and 29. The specimen collection robot system 600 does not include switches on an operation handle 621, unlike the fifth embodiment in which the switches 521a are provided on the operation handle 521. The same or similar configurations as those of the first, second, third, fourth, or fifth embodiment are denoted by the same reference numerals, and detailed description thereof is omitted.

As shown in FIG. 28, the specimen collection robot system 600 according to the sixth embodiment includes a slave robot 10, a master robot 620, an imager 30, a display 40, and a controller 650. The specimen collection robot system 600 is an example of a "robot system" in the claims.

The master robot 620 includes an operator 620a. As shown in FIG. 29, the operator 620a includes an operation handle 621. The operator 620a has substantially the same configuration as the operator 520a according to the fifth embodiment except for the operation handle 621. The operator 620a is an example of a "fourth operator" in the claims. The operation handle 621 is an example of a "fifth handle" in the claims.

The operation handle 621 is operated by one hand (the right hand in FIG. 28) of an operation person O. The operation handle 621 is provided to move a hand 12 straight ahead and move the hand 12 rotationally. Specifically, the operation handle 621 is provided to move the hand 12 straight ahead in a forward-rearward direction (A1 direction), a right-left direction (A2 direction), and an upward-downward direction (A3 direction) and rotationally move the hand 12 in a first rotation direction (C1 direction), a second rotation direction (C2 direction), and a third rotation direction (C3 direction).

In the sixth embodiment, the operation handle 621 does not include switches (the switches 521a according to the fifth embodiment). The operation handle 621 has a substantially cylindrical shape. That is, the operation handle 621 has an elongated substantially cylindrical shape like a specimen collection member 101. The operation handle 621 has a diameter and a length corresponding to the specimen collection member 101 having an elongated cylindrical shape.

In the sixth embodiment, the controller 650 controls at least the straight-ahead movement of the hand 12 and a portion of the rotational movement of the hand 12 independently of each other by dividing an operation system when a specimen is collected from a subject S by the specimen collection member 101 held by the hand 12. Specifically, the controller 650 controls at least the straight-ahead movement of the hand 12 and the rotational movement of the specimen collection member 101 of the hand 12 independently of each other by dividing the operation system.

In the sixth embodiment, the controller 650 controls at least the straight-ahead movement of the hand 12 and the rotational movement of the specimen collection member 101 of the hand 12 independently of each other by performing a control based on an operation on the operation handle 621 of the operator 620a to divide the operation system. Specifically, the controller 650 invalidates (sets to zero) operation command values (an operation command value for the straight-ahead movement of the hand 12 in the forward-rearward direction (A1 direction), an operation command value for the straight-ahead movement of the hand 12 in the right-left direction (A2 direction), an operation command value for the straight-ahead movement of the hand 12 in the upward-downward direction (A3 direction), an operation command value for the hand 12 in the second rotation direction (C2 direction), and an operation command value for the rotational movement of the hand 12 in the third rotation direction (C3 direction)) other than an operation command value for the rotational movement of the hand 12 in the first rotation direction (C1 direction) and validates only the operation command value for the rotational movement of the hand 12 in the first rotation direction (C1 direction) when an operation corresponding to the rotational movement of the specimen collection member 101 of the hand 12 is performed on the operation handle 621, and controls the movement of the hand 12.

The remaining configurations of the sixth embodiment are similar to those of the first, second, third, fourth, or fifth embodiment.

### Advantages of Sixth Embodiment

According to the sixth embodiment, the following advantages are achieved.

According to the sixth embodiment, as described above, the specimen collection robot system 600 includes the controller 650 configured or programmed to control the straight-ahead movement of the hand 12 and at least a portion of the rotational movement of the hand 12 independently of each other by dividing the operation system when a specimen is collected from the subject S by the specimen collection member 101 held by the hand 12. Accordingly, it is possible to improve the operability when the hand 12 of the slave robot 10 is remotely controlled by the master robot 620 to perform a procedure on the subject S (collect a specimen from the subject S), similarly to the first to fifth embodiments.

According to the sixth embodiment, as described above, the master robot 620 includes the operator 620a to which the straight-ahead movement and the rotational movement of the hand 12 are assigned. The operator 620a includes the operation handle 621 operated by one hand of the operation person O and having a substantially cylindrical shape without a switch. Furthermore, the controller 650 is configured or programmed to control the straight-ahead movement of the hand 12 and the rotational movement of the specimen collection member 101 of the hand 12 independently of each other by performing a control based on an operation on the operation handle 621 to divide the operation system. Accordingly, the straight-ahead movement of the hand 12 and the rotational movement of the specimen collection member 101 of the hand 12 can be controlled independently of each other based on an operation on the operation handle 621, and thus erroneous operations for the straight-ahead movement of the hand 12 and the rotational movement of the specimen collection member 101 of the hand 12 can be easily reduced or prevented. Consequently, unintended and erroneous movement of the hand 12 caused by an erroneous operation can be easily reduced or prevented. Furthermore, the operation handle 621 has a substantially cylindrical shape such that the specimen collection member 101 having a substantially cylindrical shape and the operation handle 621 can have similar shapes, and thus the operational feeling of the operation handle 621 can be brought closer to the operational feeling when the specimen collection member 101 is actually operated. Consequently, discomfort in the operability of the operation handle 621 can be reduced, and thus an erroneous operation can be reduced or prevented.

The remaining advantages of the sixth embodiment are similar to those of the first, second, third, fourth, or fifth embodiment.

### Modified Examples

The embodiments disclosed this time must be considered as illustrative in all points and not restrictive. The scope of the present disclosure is not shown by the above description of the embodiments but by the scope of claims for patent, and all modifications (modified examples) within the meaning and scope equivalent to the scope of claims for patent are further included.

For example, while the example in which the robot system is a robot system that collects a specimen from a subject using the specimen collection member has been shown in each of the aforementioned first to sixth embodiments, the present disclosure is not limited to this. In the present disclosure, the robot system may be a robot system that performs a procedure other than specimen collection on the subject (person to be treated). For example, the robot system may be a robot system that examines a subject (person to be treated) using a medical inspection instrument (treatment member).

While the example in which the slave robot is a vertical articulated robot has been shown in each of the aforementioned first to sixth embodiments, the present disclosure is not limited to this. For example, the slave robot may be a robot such as a horizontal articulated robot or a dual-arm robot.

While the example in which the straight-ahead movement of the hand and at least the rotational movement of the specimen collection member of the hand are controlled independently of each other by dividing the operation system has been shown in each of the aforementioned first to sixth embodiments, the present disclosure is not limited to this. For example, the straight-ahead movement of the hand and the rotational movement of the hand (such as the rotational movement of the hand in the second rotation direction and the rotational movement of the hand in the third rotation direction) other than the rotational movement of the specimen collection member of the hand may be controlled independently of each other by dividing the operation system.

While the example in which a control is performed to convert the arcuate movement of the hand into the linear movement has been shown in the aforementioned first embodiment, the present disclosure is not limited to this. For example, also in the configurations of the second to sixth embodiments, a control may be performed to convert the arcuate movement of the hand into the linear movement.

While the example in which the operation handle of the first operator to move the hand straight ahead is a movable grip handle supported by the arm, and the operation handle of the second operator to rotationally move the hand is a joystick has been shown in the aforementioned first embodiment, the present disclosure is not limited to this. For example, the operation handle of the first operator to move the hand straight ahead may be a joystick, and the operation handle of the second operator to rotationally move the hand may be a movable grip handle supported by the arm. Alternatively, both the operation handle of the first operator and the operation handle of the second operator may be joysticks or movable grip handles.

While the example in which the second operator includes the foot pedal to rotationally move the specimen collection member of the hand has been shown in the aforementioned second embodiment, the present disclosure is not limited to this. For example, the second operator may include a foot pedal to rotationally move the hand in the second rotation direction and a foot pedal to rotationally move the hand in the third rotation direction.

While the example in which the movement of the hand in the forward-rearward direction, the right-left direction, the upward-downward direction, the first rotation direction, the second rotation direction, and the third rotation direction is enabled when the switches are turned on, and the movement of the hand other than the movement of the hand in the first rotation direction is disabled and only the movement of the hand in the first rotation direction is enabled when the switches are turned off has been shown in the aforementioned fifth embodiment, the present disclosure is not limited to this. For example, the movement of the hand in the forward-rearward direction, the right-left direction, the upward-downward direction, the first rotation direction, the second rotation direction, and the third rotation direction may be enabled when the switches are turned off, and the movement of the hand other than the movement of the hand in the first rotation direction may be disabled and only the movement of the hand in the first rotation direction may be enabled when the switches are turned on.

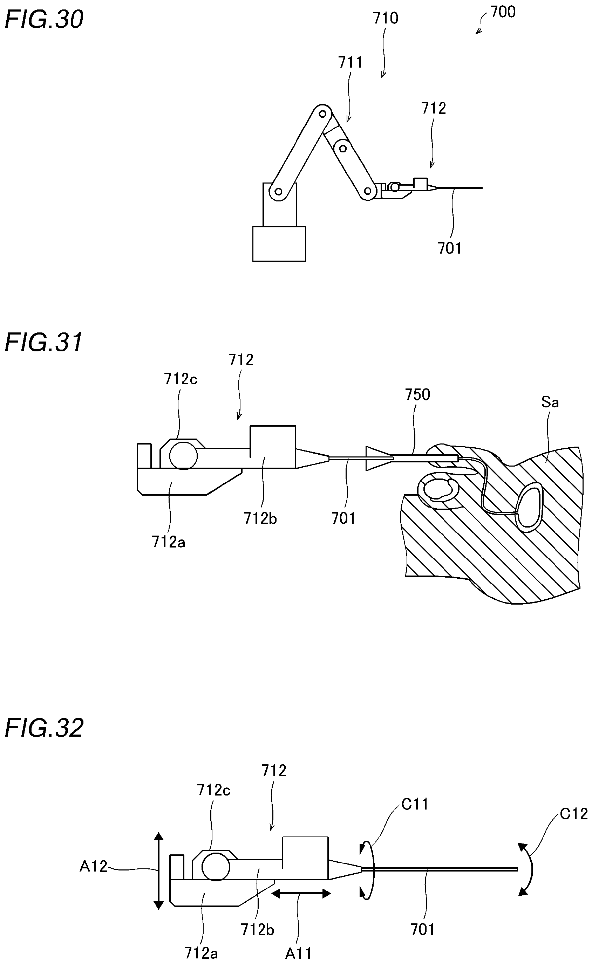

While the example in which the present disclosure is applied to the robot system that collects a specimen from a subject using the specimen collection member has been shown in each of the aforementioned first to sixth embodiments, the present disclosure is not limited to this. The present disclosure may be applied to a robot system 700 that performs ureteroscopic surgery on a person to be treated Sa, as shown in FIGS. 30 to 32, for example. The robot system 700 includes a slave robot 710, a master robot similar to any of the master robots according to the first to sixth embodiments, a display, and a controller similar to any of the controllers according to the first to sixth embodiments.

The slave robot 710 performs a procedure to perform ureteroscopic surgery on the person to be treated Sa (patient) to crush a urinary calculus using a treatment member 701. The treatment member 701 is a bendable flexible tube. The slave robot 710 inserts the treatment member 701 into the urethra of the person to be treated Sa through a ureteral access sheath 750 inserted in advance into the urethra of the person to be treated Sa by a medical staff, and images the inside of the urinary tract of the person to be treated Sa, such as the urethra and ureter. The captured video is displayed on the display. An operation person operates the slave robot 710 using the master robot to perform ureteroscopic surgery on the person to be treated Sa while checking the real-time video of the person to be treated Sa displayed on the display. In the ureteroscopic surgery, a calculus site of the person to be treated Sa is specified, the calculus is crushed by radiating laser light with a pair of laser forceps, and the calculus is retrieved with a pair of basket forceps, for example.

The slave robot 710 is a vertical articulated robot. The slave robot 710 includes an arm 711 and a hand 712 attached to the tip end of the arm 711. The arm 711 has a plurality of joints. Each of the plurality of joints of the arm 711 includes a drive such as a servomotor and a position detector such as an encoder. The hand 712 holds the treatment member 701, which is a bendable flexible tube. The hand 712 includes a hand base 712a, a ureteroscope main body 712b, and a motor 712c. The hand base 712a is attached to the tip end of the arm 711. The ureteroscope main body 712b can image the inside of the urinary tract of the person to be treated Sa. A pair of laser forceps for crushing a urinary calculus of the person to be treated Sa or a pair of basket forceps for retrieving the urinary calculus can be inserted into the ureteroscope main body 712b. The ureteroscope main body 712b holds the treatment member 701. The motor 712c rotates the ureteroscope main body 712b in a C12 direction described below.

The hand 712 is operated in a plurality of directions. Specifically, the hand 712 is moved straight ahead in a forward-rearward direction (All direction) and in an upward-downward direction (A12 direction), and is rotationally moved in a first rotation direction (C11 direction) about a first rotation axis extending in the forward-rearward direction and in a second rotation direction (C12 direction) about a second rotation axis extending in a right-left direction. The forward-rearward direction, the right-left direction, the upward-downward direction, the first rotation direction, and the second rotation direction are directions with respect to the hand 712. When the treatment member 701 is inserted into the urethra of the person to be treated Sa, the hand 712 is moved straight ahead in a forward direction.

Although the detailed description is omitted, the controller is configured or programmed to control the straight-ahead movement of the hand 712 and at least a portion of the rotational movement of the hand 712 independently of each other by dividing an operation system when a procedure is performed on the person to be treated Sa using the treatment member 701 held by the hand 712, similarly to any of the first to sixth embodiments described above. Specifically, the controller is configured or programmed to control the straight-ahead movement of the hand 712 and at least a portion of the rotational movement of the hand 712 independently of each other by dividing the operation system by a plurality of operators, switches, or rotational movement automation when a procedure is performed on the person to be treated Sa using the treatment member 701 held by the hand 712, similarly to any of the first to sixth embodiments described above.

### Description of Reference Numerals

10, 710: slave robot
12, 712: hand
20, 220, 320, 420, 520, 620: master robot
20a: first operator (first operator, fifth operator)
20b: second operator (second operator)
21: operation handle (first handle, sixth handle)
27: operation handle (second handle)
50, 250, 350, 450, 550, 650: controller
100, 200, 300, 400, 500, 600, 700: specimen collection robot system (robot system)
101: specimen collection member (treatment member)
220a: first operator (first operator)
220b: second operator (second operator)
221: operation handle (third handle)
227: foot pedal
520a: operator (third operator)
521: operation handle (fourth handle)
521a: switch
620a: operator (fourth operator)
621: operation handle (fifth handle)
701: treatment member
O: operation person
S: subject (person to be treated)
Sa: person to be treated

## Claims

1. A robot system comprising:
a slave robot including a hand to hold a treatment member and operable to perform a procedure on a person to be treated using the treatment member held by the hand;
a master robot operated by an operation person to remotely control the slave robot; and
a controller configured or programmed to control straight-ahead movement of the hand and at least a portion of rotational movement of the hand independently of each other by dividing an operation system when the procedure is performed on the person to be treated using the treatment member held by the hand.

2. The robot system according to claim 1,
wherein the controller is configured or programmed to control the straight-ahead movement of the hand and at least rotational movement of the treatment member of the hand as the rotational movement of the hand independently of each other by dividing the operation system.

3. The robot system according to claim 1,
wherein
the master robot includes a first operator to which the straight-ahead movement of the hand is assigned, and a second operator to which at least the portion of the rotational movement of the hand is assigned; and
the controller is configured or programmed to control the straight-ahead movement of the hand and at least the portion of the rotational movement of the hand independently of each other by controlling the straight-ahead movement of the hand based on an operation on the first operator by the operation person and at least the portion of the rotational movement of the hand based on an operation on the second operator by the operation person to divide the operation system.

4. The robot system according to claim 3,
wherein
the first operator includes a first handle operated by one hand of the operation person to move the hand straight ahead in a forward-rearward direction, a right-left direction, and an upward-downward direction with respect to the hand;
the second operator includes a second handle operated by the other hand of the operation person to rotationally move the hand in a first rotation direction about a first rotation axis extending in the forward-rearward direction, a second rotation direction about a second rotation axis extending in the right-left direction, and a third rotation direction about a third rotation axis extending in the upward-downward direction; and
the controller is configured or programmed to control the straight-ahead movement of the hand in the forward-rearward direction, the right-left direction, and the upward-downward direction based on the operation on the first handle, and control the rotational movement of the hand in the first rotation direction, the second rotation direction, and the third rotation direction based on the operation on the second handle.

5. The robot system according to claim 3,
wherein
the first operator includes a third handle operated by one hand of the operation person to perform the straight-ahead movement of the hand and the rotational movement of the hand other than rotational movement of the treatment member of the hand;
the second operator includes a foot pedal operated by a foot of the operation person to perform the rotational movement of the treatment member of the hand; and
the controller is configured or programmed to control the straight-ahead movement of the hand and the rotational movement of the hand other than the rotational movement of the treatment member of the hand based on an operation on the third handle, and control the rotational movement of the treatment member of the hand based on an operation on the foot pedal.

6. The robot system according to claim 1,
wherein the controller is configured or programmed to control the straight-ahead movement of the hand and rotational movement of the treatment member of the hand independently of each other by automatically rotationally moving the treatment member of the hand without an operation for the rotational movement of the treatment member of the hand by the operation person to divide the operation system.

7. The robot system according to claim 6,
wherein the controller is configured or programmed to always automatically rotationally move the treatment member of the hand without the operation for the rotational movement of the treatment member of the hand by the operation person.

8. The robot system according to claim 6,
wherein the controller is configured or programmed to automatically rotationally move the treatment member of the hand based on the treatment member being inserted into the person to be treated to a predetermined depth without the operation for the rotational movement of the treatment member of the hand by the operation person.

9. The robot system according to claim 1,
wherein
the master robot includes a third operator to which the straight-ahead movement and the rotational movement of the hand are assigned;
the third operator includes a fourth handle operated by one hand of the operation person;
the fourth handle includes a switch to restrict movement of the hand other than rotational movement of the treatment member of the hand and enable the rotational movement of the treatment member of the hand; and
the controller is configured or programmed to control the straight-ahead movement of the hand and the rotational movement of the treatment member of the hand independently of each other by performing a control based on an operation on the fourth handle and an operation on the switch to divide the operation system.

10. The robot system according to claim 1,
wherein
the master robot includes a fourth operator to which the straight-ahead movement and the rotational movement of the hand are assigned;
the fourth operator includes a fifth handle operated by one hand of the operation person and having a substantially cylindrical shape without a switch; and
the controller is configured or programmed to control the straight-ahead movement of the hand and rotational movement of the treatment member of the hand independently of each other by performing a control based on an operation on the fifth handle to divide the operation system.

11. The robot system according to claim 1,
wherein
the master robot includes a fifth operator to which the straight-ahead movement of the hand is assigned;
the fifth operator includes a sixth handle operated by one hand of the operation person; and
the controller is configured or programmed to perform a control to convert arcuate movement of the hand into linear movement based on an operation on the sixth handle by the operation person when the sixth handle is operated along an arcuate trajectory by the operation person.

12. A robot system comprising:
a slave robot including a hand to hold a treatment member and operable to perform a procedure on a person to be treated using the treatment member held by the hand;
a master robot operated by an operation person to remotely control the slave robot; and
a controller configured or programmed to control straight-ahead movement of the hand and at least a portion of rotational movement of the hand independently of each other by dividing an operation system by a plurality of operators, a switch, or rotational movement automation when the procedure is performed on the person to be treated using the treatment member held by the hand.
